# EUROPÄISCHE PATENTSCHRIFT

(11) **EP 1 940 838 B1**
(45) Veröffentlichungstag und Bekanntmachung des Hinweises auf die Patenterteilung: **22.08.2012**
(21) Anmeldenummer: 06806215.7
(22) Anmeldetag: 12.10.2006
(51) Int. Cl.: C07D 471/04, C07D 487/04, A61K 31/437, A61K 31/519, A61P 3/00, A61P 9/00

(54) **TRIAZOLOPYRIDIN-DERIVATE ALS INHIBITOREN VON LIPASEN UND PHOSPHOLIPASEN**
TRIAZOLOPYRIDINE DERIVATIVES AS INHIBITORS OF LIPASES AND PHOSPHOLIPASES
DERIVES DE TRIAZOLOPYRIDINE UTILISES COMME INHIBITEURS DE LIPASES ET DE PHOSPHOLIPASES

(30) Priorität: 19.10.2005 DE 102005049954
(43) Veröffentlichungstag der Anmeldung: 09.07.2008
(73) Patentinhaber: SANOFI, 75008 Paris (FR)
(72) Erfinder: PETRY, Stefan, 65926 Frankfurt am Main (DE); ZOLLER, Gerhard, 65926 Frankfurt am Main (DE); MÜLLER, Günter, 65926 Frankfurt am Main (DE); BARINGHAUS, Karl-Heinz, 65926 Frankfurt am Main (DE); HEUER, Hubert, 65926 Frankfurt am Main (DE)
(74) Vertreter: Löwrick, Oliver
(86) Internationale Anmeldenummer: PCT/EP2006/009856
(87) Internationale Veröffentlichungsnummer: WO 2007/045392

(56) Entgegenhaltungen:
- WO-A-02/055083
- WO-A-2004/035550
- WO-A-2005/073199
- DATABASE CA [Online] CHEMICAL ABSTRACTS SERVICE, COLUMBUS, OHIO, US; SIMONS, MICHAEL J. ET AL: "Development restrainer precursors for photographic elements" XP002411131 gefunden im STN Database accession no. 1980:485059 & RESEARCH DISCLOSURE , 191, 122-5 (NO. 19126) CODEN: RSDSBB; ISSN: 0374-4353, 1980,

## Beschreibung

Die vorliegende Erfindung betrifft Triazolopyridin-derivate der allgemeinen Formel I, deren pharmazeutisch anwendbare Salze und deren Verwendung als Arzneistoffe.

Benzotriazole sind bereits aus den verschiedensten Gebieten bekannt, wie z.B. der Fotochemie (US 4,255,510, Kodak) oder als Orexin Antagonisten (WO 02/090355, SKB). Ferner ist die Synthese zur Herstellung von Benzotriazolen von Katritzky et al. in J. Org. Chem. 1997, 62, 4155-4158 beschrieben.
In der WO 02/055083 werden Triazolopyrimidin-derivate als purinergische Rezeptor Antagonisten beschrieben.
Indazole als Inhibitoren der Hormonsensitiven Lipase sind in der W02005/073199 beschrieben.
Bekannt sind ferner Carbamate als Lipase-Inhibitoren wie z.B. Shamkant Patkar et al. in Paul Woolley, Steffen B. Petterson (ed), Lipase (1994) 207-227, WO 03/051842 oder WO 2004/035550.

Aufgabe der vorliegenden Erfindung ist die Bereitstellung von Verbindungen, die eine Hemmung der hormonsensitiven Lipase oder der endothelialen Lipase bewirken.

Gegenstand der Erfindung sind Triazolopyridin-derivate der allgemeinen Formel I 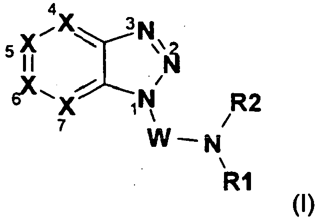 wobei bedeuten:
- X: gleich oder verschieden =C(-R)- oder =N-, wobei ein X für =N- steht;
- W: -(C=O)-, -(S=O)-, -(SO₂)-;
- R: gleich oder verschieden Wasserstoff, Halogen, (C₁-C₆)-Alkyl, (C₁-C₃)-Haloalkyl, (C₁-C₃)-Alkyloxy-(C₁-C₃)-Alkylen, Hydroxy, Phenoxy, Amino, (C₁-C₆)-Alkylamino, di-(C₂-C₁₂)-Alkylamino, Cyano, Nitro, COOR3, mono-(C₁-C₆)-Alkylaminocarbonyl, di-(C₂-C₈)-Alkylaminocarbonyl, (C₁-C₆)-Alkylmercapto, (C₁-C₆)-Alkylsulfinyl, (C₁-C₆)-Alkylsulfonyl, Aminosulfonyl, Pentafluorsulfanyl, Aryl, Heteroaryl, (C₃-C₁₂)-Cycloalkyl, (C₁-C₆)-Alkylcarbonyl, O-CO-NR3R3a, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR3R3a oder unsubstituiertes oder ein oder mehrfach durch F substituiertes (C₁-C₆)-Alkyloxy;
- R1: (C₅-C₁₆)-Alkyl, (C₅-C₁₂)-Cycloalkyl, Y-Aryl, Y-Heteroaryl, Y-(C₅-C₁₂)-Cycloalkyl, Bicyclus, wobei Aryl, Heteroaryl, Cycloalkyl oder Bicyclus durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkyloxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein oder mehrfach substituiert sein kann;
- Y: (C₁-C₃)-Alkylen, welches durch Halogen, (C₁-C₂)-Alkyl oder Hydroxy ein-oder mehrfach substituiert sein kann;
- R2: Wasserstoff, (C₄-C₁₂)-Alkyl; oder

- R1 und R2: zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, -SO-, -SO₂-, ersetzt sein können, mit der Maßgabe, dass zwei Einheiten aus der Reihe -O-, -S-, -SO-, -SO₂- nicht benachbart sein dürfen;
- R3, R3a: gleich oder verschieden Wasserstoff, (C₁-C₆)-Alkyl, Benzyl;
- R4, R5: gleich oder verschieden (C₁-C₁₂)-Alkyl, (C₁-C₆)-Alkyloxy, Halogen, (C₁-C₃)-Haloalkyl, (C₁-C₃)-Haloalkyloxy, COOR3, Nitro, Cyano, Aryl, Heterocyclus, (C₃-C₁₂)-Cycloalkyl, (C₁-C₃)-Alkylen-Aryl, (C₁-C₃)-Alkylen-Heterocyclus, (C₁-C₃)-Alkylen-Cycloalkyl, wobei Aryl, Heterocyclus oder Cycloalkyl ein oder mehrfach durch bevorzugt Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, NR3R3a substituiert sein kann;
die tautomeren Formen der Verbindungen sowie deren physiologisch verträgliche Salze,
mit der Maßgabe, dass die Verbindung der Formel I mit den Bedeutungen X in 4, 5 und 6-Position =C(-R)-, in 7-Position =N-, W ist -(C=O)-, R ist H und R1 und R2 bilden ein Morpholin ausgenommen ist.

Bevorzugt sind Verbindungen der Formel I, worin
- X: gleich oder verschieden =C(-R)- oder =N-, wobei ein X für =N- steht;
- W: -(C=O)-;
bedeutet.

Besonders bevorzugt sind Verbindungen der Formel I, worin
- X: gleich oder verschieden =C(-R)- oder =N-, wobei ein X für =N- steht;
- W: -(C=O)-;
- R: gleich oder verschieden Wasserstoff, Halogen, (C₁-C₆)-Alkyl, Trifluormethyl, (C₁-C₃)-Alkyloxy-(C₁-C₃)-Alkylen, Hydroxy, Phenoxy, Amino, (C₁-C₆)-Alkylamino, di-(C₂-C₁₂)-Alkylamino, Cyano, Nitro, COOR3, mono-(C₁-C₆)-Alkylaminocarbonyl, di-(C₂-C₈)-Alkylaminocarbonyl, (C₁-C₆)-Alkylsulfonyl, Aminosulfonyl, Pentafluorsulfanyl, Phenyl, (C₁-C₆)-Alkylcarbonyl, O-CO-NR3R3a, O-CO-(C₁-C₆)-Alkylen-CO-O-(C₁-C₆)-Alkyl, O-C0-(C₁-C₆)-Alkylen-CO-OH, O-CO-(C₁-C₆)-Alkylen-CO-NR3R3a oder unsubstituiertes oder ein oder mehrfach durch F substituiertes (C₁-C₆)-Alkyloxy;
- R1: (C₆-C₁₂)-Alkyl, (C₅-C₁₂)-Cycloalkyl, Y-Phenyl, Y-Heteroaryl, Bicyclus, wobei Phenyl, Heteroaryl, Cycloalkyl oder Bicyclus durch bevorzugt Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkyloxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein oder mehrfach substituiert sein kann;
- Y: -CH₂-, welches durch Fluor, Methyl oder Hydroxy einfach substituiert sein kann;
- R2: Wasserstoff, (C₅-C₁₀)-Alkyl; oder
- R1 und R2: zusammen mit dem sie tragenden Stickstoffatom ein monocyclisches, gesättigtes oder teilweise ungesättigtes 5- bis 6-gliedriges Ringsystem oder ein bicyclisches gesättigtes oder teilweise ungesättigtes 9- bis 10. gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, -SO₂-, ersetzt sein können, mit der Maßgabe, dass zwei Einheiten aus der Reihe -O-, -S-, -SO₂- nicht benachbart sein dürfen;

- R3, R3a: gleich oder verschieden Wasserstoff, (C₁-C₆)-Alkyl, Benzyl;
- R4, R5: gleich oder verschieden (C₁-C₁₀)-Alkyl, (C₁-C₆)-Alkyloxy, Halogen, (C₁-C₃)-Haloalkyl, (C₁-C₃)-Haloalkyloxy, COOR3, Nitro, Cyano, Aryl, Heterocyclus, (C₃-C₇)-Cycloalkyl, (C₁-C₃)-Alkylen-Aryl, (C₁-C₃)-Alkylen-Heteroaryl, wobei Aryl, Heterocyclus oder Cycloalkyl ein oder mehrfach durch bevorzugt Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, NR3R3a substituiert sein kann;
bedeuten;
die tautomeren Formen der Verbindungen sowie deren physiologisch verträgliche Salze.

Besonders bevorzugt sind ferner Verbindungen der Formel I, worin
- X: gleich oder verschieden =C(-R)- oder =N-, wobei ein X für =N- steht;
- W: -(C=O)-;
- R: gleich oder verschieden Wasserstoff, Halogen, (C₁-C₆)-Alkyl, Trifluormethyl, Hydroxy, Amino, Cyano, COOR3, mono-(C₁-C₆)-Alkylaminocarbonyl, di-(C₂-C₈)-Alkylaminocarbonyl, (C₁-C₆)-Alkylsulfonyl, Aminosulfonyl, Pentafluorsulfanyl, (C₁-C₆)-Alkylcarbonyl, O-CO-NR3R3a, O-CO-(C₁-C₆)-Alkylen-CO-O-(C₁-C₆)-Alkyl oder unsubstituiertes oder ein oder mehrfach durch F substituiertes (C₁-C₆)-Alkyloxy;
- R1: (C₆-C₁₂)-Alkyl, Y-Phenyl, Y-Heteroaryl, Bicyclus, wobei Aryl, Heteroaryl oder Bicyclus durch bevorzugt Halogen, (C₁-C₆)-Alkyl, (C₁-C₆)-Alkyloxy, Hydroxy, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)- , Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein oder mehrfach substituiert sein kann;
- Y: -CH₂-;
- R2: Wasserstoff, (C₅-C₁₀)-Alkyl; oder
- R1 und R2: zusammen mit dem sie tragenden Stickstoffatom ein monocyclisches, gesättigtes oder teilweise ungesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, ersetzt sein können, mit der Maßgabe, dass zwei Einheiten aus der Reihe -O-, -S-, nicht benachbart sein dürfen;
- R3, R3a: gleich oder verschieden Wasserstoff, (C₁-C₆)-Alkyl, Benzyl;
- R4, R5: gleich oder verschieden (C₁-C₁₀)-Alkyl, (C₁-C₆)-Alkyloxy, Halogen, (C₁-C₃)-Haloalkyl, (C₁-C₃)-Haloalkyloxy, COOR3, Nitro, Cyano, Aryl, Heteroaryl, (C₃-C₇)-Cycloalkyl, (C₁-C₃)-Alkylen-Aryl, (C₁-C₃)-Alkylen-Heteroaryl, wobei Aryl, Heteroaryl oder Cycloalkyl ein oder mehrfach durch bevorzugt Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, Amino substituiert sein kann;
bedeuten;
die tautomeren Formen der Verbindungen sowie deren physiologisch verträgliche Salze.

Ganz besonders bevorzugt sind Verbindungen der Formel I, worin
- X: gleich oder verschieden =C(-R)- oder =N-, wobei ein X für =N- steht;
- W: -(C=O)-;
- R: gleich oder verschieden Wasserstoff, Halogen, (C₁-C₆)-Alkyl, Trifluormethyl, Hydroxy, Amino, Cyano, COOR3, (C₁-C₆)-Alkylsulfonyl, Aminosulfonyl, (C₁-C₆)-Alkylcarbonyl oder unsubstituiertes oder ein oder mehrfach durch F substituiertes (C₁-C₆)-Alkyloxy;
- R1: (C₆-C₁₂)-Alkyl, Y-Phenyl, wobei Phenyl durch bevorzugt Halogen, (C₁-C₆)-Alkyl, (C₁-C₆)-Alkyloxy, Hydroxy, Amino, (C₁-C₆)-Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein bis dreifach substituiert sein kann;
- Y: -CH₂-;
- R2: Wasserstoff, (C₅-C₁₀)-Alkyl; oder
- R1 und R2: zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, ersetzt sein können, mit der Maßgabe, dass zwei Einheiten aus der Reihe -O-, -S-, nicht benachbart sein dürfen;
- R3, R3a: gleich oder verschieden Wasserstoff, (C₁-C₆)-Alkyl;
- R4, R5: gleich oder verschieden (C₁-C₁₀)-Alkyl, (C₁-C₆)-Alkyloxy, Halogen, Trifluormethyl, Trifluormethoxy, COOR3, Cyano, Aryl, Cyclopropyl, (C₁-C₃)-Alkylen-Phenyl, wobei Aryl ein oder mehrfach durch bevorzugt Halogen, (C₁-C₆)-Alkyl, (C₁-C₆)-Alkyloxy, O-(C₁-C₃)-Alkylen-Phenyl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, Amino substituiert sein kann;
bedeuten;
die tautomeren Formen der Verbindungen sowie deren physiologisch verträgliche Salze.

Ganz besonders bevorzugt sind auch die Verbindungen der Formel I, worin
- NR1R2: für ein monocyclisches gesättigtes 5- bis 6-gliedriges Ringsystem steht, das in 4 Position ein Atom oder Atomglied aus der Reihe -CHR4-, - CR4R5-, -NR5-, -(C=O) enthält oder für ein bicyclisches gesättigtes oder teilweise ungesättigtes 9- bis 10 gliedriges Ringsystem, dessen einzelne Glieder des Ringsystems durch ein oder zwei Atomgruppen aus der Reihe -CHR4-, -(C=R4)-, =(C-R4)-, -NR5-, -(C=O)- ersetzt sein können.

Ganz besonders bevorzugt sind weiterhin die Verbindungen der Formel I, worin

R1 und R2 zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden, deren einzelne Glieder der Ringsysteme durch eine Atomgruppe aus der Reihe -CHR4-, -NR5, ersetzt sein können.

Eine insbesondere ganz besonders bevorzugte Ausführungsform sind Verbindungen der Formel I, worin
- X: gleich oder verschieden =C(-R)- oder =N-, wobei ein X für =N- steht;
- R: gleich oder verschieden Wasserstoff, Cl;
- NR1R2: für Piperidin steht, das in 4 Position das Atomglied -CHR4- enthält; oder für Piperazin steht, das in 4-Position das Atomglied -NR5 enthält;
- R4, R5: (C₁-C₈)-Alkyl, Phenyl, -(C₁-C₂)-Alkylen-Phenyl, Naphtyl, Pyrrolidin-2-on, Benzofuranyl, wobei Phenyl ein-, zwei- oder dreifach durch Methyl, Ethyl, t-Butyl, Methoxy, Ethoxy, F, Cl, Br, OH, CF₃, NH₂, CO-OCH₃, CO-CH₃, Phenyl, -O-Benzyl substituiert sein kann;
bedeuten.

Insbesondere besonders bevorzugt sind auch die Verbindungen der Formel I, worin
- NR1R2: für Piperidin steht, das in 4 Position das Atomglied -CHR4- enthält oder für Piperazin steht, das in 4-Position das Atomglied -NR5 enthält;
- R4, R5: Methyl oder Phenyl, welches durch Methyl, Methoxy, Ethoxy, F, Cl, Br, OH, CF₃, NH₂, CO-OCH₃, CO-CH₃, Phenyl, -O-Benzyl, ein oder zweifach substituiert sein kann; bedeuten.

Eine weitere insbesondere ganz besonders bevorzugte Ausführungsform sind Verbindungen der Formel I, worin bedeuten
- X: gleich oder verschieden =C(-R)- oder =N-, wobei ein X für =N- steht;
- R: gleich oder verschieden Wasserstoff, Cl;
- NR1R2: für Tetrahydrochinolin oder Tetrahydroisochinolin steht, welches durch Methoxy ein oder zweifach substituiert sein kann.

Besonders bevorzugt sind ferner Verbindungen der Formel I, worin
- R2: Wasserstoff;
bedeutet.

Ganz besonders bevorzugt sind auch Verbindungen der Formel I, worin
- R1: (C₆-C₁₂)-Alkyl, Y-Phenyl, wobei Phenyl durch bevorzugt Halogen, (C₁-C₆)-Alkyl, (C₁-C₆)-Alkyloxy, Hydroxy, Amino, (C₁-C₆)-Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein bis dreifach substituiert sein kann;
- Y: -CH₂-;
- R2: Wasserstoff;
bedeuten.

Eine weitere insbesondere ganz besonders bevorzugte Ausführungsform sind Verbindungen der Formel I, worin
- X: gleich oder verschieden =C(-R)- oder =N-, wobei ein X für =N- steht;
- R: gleich oder verschieden Wasserstoff, Cl;
- R1: Hexyl, Y-Phenyl, wobei Phenyl durch Methyl, bevorzugt in 2-Position, substituiert sein kann;
- Y: -CH₂-;
- R2: Wasserstoff;
bedeuten.

Ganz besonders bevorzugt sind auch die Verbindungen der Formel I, worin
- X: in Position 4, 5 und 6 für gleich oder verschieden =C(-R)-, in Position 7 für =N- steht.

Ferner sind ganz besonders bevorzugt Verbindungen der Formel I, worin
- X: gegebenenfalls in Position 4 oder 7 für =C(-R)- mit R = Wasserstoff oder =N- steht, in Position 5 oder 6 für =C(-R)- mit R nicht Wasserstoff steht.

Die Erfindung bezieht sich auf Verbindungen der Formel I, in Form ihrer Salze, Racemate, racemischen Mischungen und reinen Enantiomere sowie auf ihre Diastereomere und Mischungen davon.

Die Alkylreste in den Substituenten R, R1, R2, R3, R3a, R4, R5 können sowohl geradkettig wie verzweigt sein. Beispielhaft seien genannt: Methyl, Ethyl, n-Propyl, i-Propyl, n-Butyl, i-Butyl, t-Butyl, Pentyl, Hexyl, Octyl, Dodecyl. Halogen steht für Fluor, Chlor, Brom oder lod, besonders für Fluor oder Chlor.

Unter Haloalkyl wird ein durch Halogen ein oder mehrfach substituierter Alkylrest verstanden.

Unter einem Arylrest wird ein Phenyl-, Naphthyl- oder Biphenylrest verstanden.
Die Arylreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂, (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO- (C₁-C₆)-Aryl,
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus, SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ , NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Heterocyclus ist ein mono- oder bicyclisches Ringsystem mit 5 bis 12 Ringgliedern, worin mindestens ein Atom im Ringsystem ein Heteroatom aus der Reihe N, O und S ist. Ferner gehören auch Ringsysteme zu dieser Definition, worin der Heterocyclus mit einem Benzolkern kondensiert ist. (C₅-C₇)-Heterocyclus ist ein monozyklisches, (C₈-C₁₂)-Heterocyclus ein bicyclisches Ringsystem.
Heteroaryl ist eine Untergruppe von Heterocyclus und ist ein mono- oder bicyclisches aromatisches Ringsystem mit 5 bis 12 Ringgliedern, worin mindestens ein Atom im Ringsystem ein Heteroatom aus der Reihe N, O und S ist.
Bevorzugte Heteroarylreste sind Thienyl und Pyridyl, insbesondere Thienyl.

Geeignete "Heterocyclische Ringe" bzw. "Heterocyclische Reste" sind Azocinyl, Benzimidazolyl, Benzofuryl, Benzothienyl, Benzothiophenyl, Benzoxazolyl, Benzthiazolyl, Benztriazolyl, Benztetrazolyl, Benzisoxazolyl, Benzisothiazolyl, Benzimidazalinyl, Carbazolyl, 4aH-Carbazolyl, Carbolinyl, Chinazolinyl, Chinolinyl, 4H-Chinolizinyl, Chinoxalinyl, Chinuclidinyl, Chromanyl, Chromenyl, Cinnolinyl, Decahydrochinolinyl, 2H,6H-1,5,2-Dithiazinyl, Dihydrofuro[2,3-b]-Tetrahydrofuran, Furyl, Furazanyl, Imidazolidinyl, Imidazolinyl, Imidazolyl, 1H-Indazolyl, Indolinyl, Indolizinyl, Indolyl, 3H-Indolyl, Isobenzofuranyl, Isochromanyl, Isoindazolyl, Isoindolinyl, Isoindolyl, Isochinolinyl (Benzimidazolyl), Isothiazolyl, Isoxazolyl, Morpholinyl, Naphthyridinyl, Octahydroisochinolinyl, Oxadiazolyl, 1,2,3-Oxadiazolyl, 1,2,4-Oxadiazolyl, 1,2,5-Oxadiazolyl, 1,3,4-Oxadiazolyl, Oxazolidinyl, Oxazolyl, Oxazolidinyl, Pyrimidinyl, Phenanthridinyl, Phenanthrolinyl, Phenazinyl, Phenothiazinyl, Phenoxathiinyl, Phenoxazinyl, Phthalazinyl, Piperazinyl, Piperidinyl, Pteridinyl, Purynyl, Pyranyl, Pyrazinyl, Pyroazolidinyl, Pyrazolinyl, Pyrazolyl, Pyridazinyl, Pyridooxazole, Pyridoimidazole, Pyridothiazole, Pyridinyl, Pyridyl, Pyrimidinyl, Pyrrolidinyl, Pyrrolinyl, 2H-Pyrrolyl, Pyrrolyl, Tetrahydrofuranyl, Tetrahydroisochinolinyl, Tetrahydrochinolinyl, 6H-1,2,5-Thiadazinyl, Thiazolyl, 1,2,3-Thiadiazolyl, 1,2,4-Thiadiazolyl, 1,2,5-Thiadiazolyl, 1,3,4-Thiadiazolyl, Thienyl, Triazolyl, Tetrazolyl und Xanthenyl.
Pyridyl steht sowohl für 2-, 3- als auch 4-Pyridyl. Thienyl steht sowohl für 2- als auch 3-Thienyl. Furyl steht sowohl für 2- als auch 3-Furyl.
Umfasst sind weiterhin die entsprechenden N-Oxide dieser Verbindungen, also z.B. 1-Oxy-2-, 3- oder 4-pyridyl.

Die Heterocyclische Reste, bzw. die Heteroaromatischen Reste können ein oder mehrfach mit geeigneten Gruppen substituiert sein, wie z.B. F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂, , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Unter einem Cycloalkylrest wird ein einen oder mehrere Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.
Die Cycloalkylreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]₂. , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂ , NH-(CH₂)ₙ-Aryl, NH-(CH₂)ₙ-Heterocyclus, N((C₁-C₆)-Alkyl)(CH₂)ₙ-Aryl, N((C₁-C₆)-Alkyl)(CH₂)ₙ-Heterocyclus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyₗ)₂, SO₂-CH₃, COOH, COO-(C₁-C₆)-Alkyl, CONH₂.

Bicyclus ist ein teilweise ungesättigtes bicyclisches Ringsystem mit 8 bis 14 Ringgliedern, das ausschließlich Kohlenstoffatome als Ringglieder hat. Beispielhaft seien der Tetrahydronaphthyl-, alpha- oder beta-Tetralon-, Indanyl- oder Indan-1-on-ylrest genannt. Bevorzugte Bicyclusreste sind Tetrahydronaphtyl und Indanyl.

Die Bicyclusreste können ein oder mehrfach mit geeigneten Gruppen substituiert sein, wie z.B.: F, Cl, Br, I, CF₃, OH, OCF₃, NO₂, CN, COOH, COO(C₁-C₆)Alkyl, CONH₂, CONH(C₁-C₆)Alkyl, CON[(C₁-C₆)Alkyl]_{2.} , (C₃-C₁₀)-Cycloalkyl, (C₁-C₁₀)-Alkyl, (C₂-C₆)-Alkenyl, (C₂-C₆)-Alkinyl, O-(C₁-C₆)-Alkyl, CO-(C₁-C₆)-Alkyl, O-CO-(C₁-C₆)-Alkyl, O-CO-(C₁-C₆)-Aryl,
PO₃H₂, SO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Aryl, S-(CH₂)ₙ-Heterocyclus, SO-(C₁-C₆)-Alkyl, SO-(CH₂)ₙ-Aryl, SO-(CH₂)ₙ-Heterocyclus, SO₂-(C₁-C₆)-Alkyl, SO₂-(CH₂)ₙ-Aryl, SO₂-(CH₂)ₙ-Heterocyclus , SO₂-NH(CH₂)ₙ-Aryl, SO₂-NH(CH₂)ₙ-Heterocyclus, SO₂-N(C₁-C₆)-Alkyl)(CH₂)ₙ-Aryl, SO₂-N(C₁-C₆)-Alkyl)(CH₂)ₙ-Heterocyclus, SO₂-N((CH₂)ₙ-Aryl)₂, SO₂-N((CH₂)ₙ-(Heterocyclus)₂, NH-(CH₂)ₙ-Aryl, NH-(CH₂)ₙ-Heterocyclus, N((C₁-C₆)-Alkyl)(CH₂)-Aryl, N((C₁-C₆)-Alkyl)(CH₂)ₙ-Heterocydus,
C(NH)(NH₂), NH₂, NH-(C₁-C₆)-Alkyl, N((C₁-C₆)-Alkyl)₂, NH-CO-(C₁-C₆)-Alkyl, NH-COO-(C₁-C₆)-Alkyl, NH-CO-Aryl, NH-CO-Heterocyclus, NH-COO-Aryl, NH-COO-Heterocyclus, NH-CO-NH-(C₁-C₆)-Alkyl, NH-CO-NH-Aryl, NH-CO-NH-Heterocyclus, Aryl, O-(CH₂)ₙ-Aryl, O-(CH₂)ₙ-Heterocyclus, wobei n = 0 - 6 sein kann, wobei der Arylrest oder Heterocyclische Rest ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliche Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium-und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

### Verwendung

Die erfindungsgemäßen Verbindungen der allgemeinen Formel I besitzen eine überraschende hemmende Wirkung an der Hormon Sensitiven Lipase, HSL, einem allosterischen Enzym in Adipozyten, das durch Insulin gehemmt wird und für den Abbau von Fetten in Fettzellen und damit für die Überführung von Fettbestandteilen in die Blutbahn verantwortlich ist. Eine Hemmung dieses Enzyms entspricht also einer insulinähnlichen Wirkung der erfindungsgemäßen Verbindungen, die letztlich zu einer Verminderung von freien Fettsäuren im Blut und von Blutzucker führt. Sie können also eingesetzt werden bei Entgleisungen des Stoffwechsels wie zum Beispiel beim nicht insulinabhängigen Diabetes Mellitus, beim diabetischen Syndrom und bei direkter Schädigung des Pankreas.
Die erfindungsgemäßen Verbindungen der allgemeinen Formel I, besonders diejenigen, in denen R2 Wasserstoff ist, können eine hemmende Wirkung auf die endotheliale Lipase (EL) besitzen. Das antiatheroskleotisch wirksame HDL ist das bevorzugte Substrat für EL. Eine Senkung des HDL-Spiegels führt zur Progression von Atherosklerose und ihrer Folgeerkrankungen wie Metabolisches Syndrom und Koronare Herzerkrankungen. Eine Hemmung der EL sollte somit zur Vorbeugung von atherosklerotischen Erkrankungen führen.
Die erfindungsgemäßen Verbindungen der Formel I können auch eine hemmende Wirkung auf die Triglyceridlipase aufweisen.

Weiterhin wurde gefunden, dass die hemmende Wirkung der erfindungsgemäßen Verbindungen der allgemeinen Formel I selektiv gegenüber anderen Lipasen ist.

Besonders geeignet sind solche Verbindungen zur Behandlung und/oder Prävention von
1. - Störungen des Fettsäurestoffwechsels und Glucoseverwertungsstörungen
2. Störungen der Insulin-Sensitivität von Muskel-, Fett- und Leberzellen (InsulinResistenz) - Metabolisches Syndrom
3. Diabetes mellitus, insbesondere Typ 2 Diabetes einschließlich der Verhinderung der damit verbundenen Folgeerkrankungen.
   Besondere Aspekte sind dabei die
   - Hyperglykämie,
   - Verbesserung der Insulinresistenz,
   - Verbesserung der Glucose-Toleranz,
   - Schutz der ß-Zellen der Bauchspeicheldrüse
   - Verhinderung makro- und mikrovaskulärer Erkrankungen
4. Dyslipidämien und deren Folgen, wie z.B. Atherosklerose, koronare Herzkrankheit, zerebrovaskuläre Erkrankungen etc, insbesondere solche (aber nicht beschränkt auf), die durch einen oder mehrerer folgender Faktoren charakterisiert sind:
   - hohe Plasma-Triglycerid-, hohe postprandiale Plasma-Triglycerid-Konzentrationen
   - niedrige HDL-Cholesterin Konzentration
   - niedrige ApoA Lipoprotein-Konzentrationen
   - hohe LDL-Cholesterin Konzentrationen
   - kleine dichte LDL-Cholesterin Partikel
   - hohe ApoB Lipoprotein-Konzentrationen
5. Verschiedenen andere Zuständen, die mit dem Metabolischen Syndrom assoziert sein können, sind wie:
   - Adipositas (Fettsucht), einschließlich abdominale Adipositas
   - Thrombosen, Stadien von Hyperkoagulabilität und Thromboseneigung (arteriell und venös)
   - Hoher Blutdruck
   - Herzinsuffizienz, wie z.B. (aber nicht beschränkt auf) bei Zustand nach Myokardinfarkt, hypertensive Herzerkrankung oder Kardiomyopathie
6. weitere Krankheiten oder Zustände bei welchen zum Beispiel entzündliche Reaktionen oder die Zelldifferenzierung eine Rolle spielt sind:
   - Atherosklerose wie z.B. (aber nicht beschränkt auf) Koronarsklerose einschl. Angina pectoris oder Herzinfarkt, Hirnschlag
   - Vaskuläre Restenose oder Reverschluß
   - Chronisch entzündliche Darmerkrankungen, wie z.B. Morbus Crohn und Colitis ulcerosa
   - Pankreatitis
   - Andere entzündliche Zustände
   - Retinopathie
   - Fettzell-Tumore (adipose cell tumors)
   - Fettzell-Karzinome, wie z.B. Liposarkome
   - solide Tumoren und Neoplasien, wie z.B. (aber nicht beschränkt auf) Karzinome des Magen-Darm Traktes, der Leber, der Gallenwege und des Pankreas, endokrine Tumore, Karzinome der Lunge, der Niere und harnableitenden Organe, des Genitaltraktes, Prostata-Karzinome etc.
   - akute und chronische myeloprolifeative Erkrankungen und Lymphome
   - Angiogenese
   - Neurodegenerative Erkrankungen
   - Alzheimersche Krankheit
   - Multiple Sklerose
   - Morbus Parkinson
   - Erythemato-squamöse Dermatosen, wie z.B. Psoriasis (Schuppenflechte)
   - Akne vulgaris
   - Andere Hautkrankheiten und dermatologische Zustände, die durch PPAR moduliert werden
   - Ekzeme und Neurodermitis
   - Dermatitiden, wie z.B. seborrhoische Dermatitis oder Lichtdermatitis
   - Keratitis und Keratosen, wie z.B. seborrhoische Keratosen, senile Keratosen, aktinische Keratose, photo-induzierte Keratosen oder Keratosis follicularis
   - Keloide und Keloid-Prophylaxe
   - Warzen, einschließlich Kondylomata oder Kondylomata acuminata
   - Human papilloma viral (HPV) Infektionen, wie z.B. venerische Papillomata, virale Warzen, wie z.B. Molluscum contagiosum, Leukoplakie
   - Papulöse Dermatosen, wie z.B. Lichen planus
   - Hautkrebs, wie z.B. Basalzellkarzinome, Melanome oder kutane T-Zell Lymphome
   - Lokalisierte, benigne epidermale Tumore, wie z.B. Keratoderma, epidermale Naevi
   - Frostbeulen
   - Hoher Blutdruck
   - Syndrom X
   - Syndrom der polyzystischen Ovarien (PCOS)
   - Asthma
   - Osteoarthritis
   - Lupus erythematodes (LE) oder entzündliche rheumatische Erkrankungen, wie z.B. Rheumatoide Arthritis
   - Vaskulitis
   - Auszehrung (Kachexie)
   - Gicht
   - Ischämie/Reperfusions Syndrom
   - Akutes respiratorisches Distress Syndrom (ARDS) ("Schocklunge")

### Galenik

Die Menge einer erfindungsgemäßen Verbindung, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg bis 50 mg) pro Tag pro Kilogramm Körpergewicht, z.B. 3 -10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 0.05 bis 1000 mg, typischerweise von 0,5 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer erfindungsgemäßer Verbindungen. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Polyvinylacetatphthalat, Hydroxypropylmethylcellulosephthalat und anionische Polymere von Methacrylsäure und Methacrylsäuremethylester.

Geeignete pharmazeutische Zubereitungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch Tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mitteln in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, welche die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglycole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.

Die Verbindungen der Formeln I zeichnen sich durch günstige Wirkungen auf Stoffwechselstörungen aus. Sie beeinflussen den Fett- und Zuckerstoffwechsel positiv, sie senken insbesondere den Triglyceridspiegel und sind zur Prävention und Behandlung von Typ II Diabetes und Arteriosklerose sowie deren vielfältigen Folgeerkrankungen geeignet.

### Kombinationen mit anderen Medikamenten

Die erfindungsgemäßen Verbindungen können alleine oder in Kombination mit einem oder mehreren weiteren pharmakologischen Wirkstoffen verabreicht werden. Insbesondere können die erfindungsgemäßen Verbindungen mit Wirkstoffen, die eine ähnliche pharmakologische Wirkung wie sie selbst aufweisen, verabreicht werden. Beispielsweise können sie in Kombination mit Wirkstoffen, die eine günstige Wirkungen auf Stoffwechselstörungen bzw. häufig damit assoziierte Erkrankungen haben, verabreicht werden. Beispiele für solche Medikamente sind
1. blutzuckersenkende Medikamente, Antidiabetika,
2. Wirkstoffe zur Behandlung von Dyslipidämien,
3. antiatherosklerotische Medikamente,
4. Mittel gegen Obesitas,
5. entzündungshemmende Wirkstoffe
6. Wirkstoffe zur Behandlung von malignen Tumoren
7. antithrombotische Wirkstoffe
8. Wirkstoffe zur Behandlung von Bluthochdruck
9. Wirkstoffe zur Behandlung von Herzinsuffizienz und
10. Wirkstoffe zur Behandlung und/oder Prävention von durch Diabetes verursachten bzw. mit Diabetes assoziierten Komplikationen.
11. Wirkstoffe zur Behandlung von neurodegenerativen Krankheiten
12. Wirkstoffe zur Behandlung von Krankheiten des Zentralen Nervensystems
13. Wirkstoffe zur Behandlung von Drogen-, Nikotin- und Alkoholabhängigkeit
14. Schmerzmittel

Sie lassen sich mit den erfindungsgemäßen Verbindungen der Formel I insbesondere zur synergistischen Wirkungsverbesserung kombinieren. Die Verabreichung der Wirkstoffkombination kann entweder durch getrennte Verabreichung der Wirkstoffe an den Patienten oder in Form von Kombinationsprodukten, bei denen mehrere Wirkstoffe in einer pharmazeutischen Zubereitung vorhanden sind, erfolgen.

Als weitere Wirkstoffe für die Kombinationspräparate sind insbesondere geeignet:
Alle Antidiabetika, die in der Roten Liste 2006, Kapitel 12 genannt sind; alle Abmagerungsmittel/Appetitzügler, die in der Roten Liste 2006, Kapitel 1 genannt sind;
alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder solche, wie sie in W02005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn™ (Generex Biotechnology), GLP-1-Derivate wie z.B. Exenatide, Liraglutide oder diejenigen die in WO 98/08871 oder W02005027978 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.
Die Wirkstoffe umfassen vorzugsweise
Sulfonylharnstoffe,
Biguanide,
Meglitinide,
Oxadiazolidindione,
Thiazolidindione,
Glukosidase-Inhibitoren,
Hemmstoffe der Glykogen Phosphorylase,
Glukagon-Antagonisten,
Glukokinase Aktivatoren,
Inhibitoren der Fructose-1,6-bisphosphatase,
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. diejenigen, die in WO 97/26265 und WO 99/03861 von Novo Nordisk A/S offenbart wurden,
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11ß-HSD1,
Inhibitoren der Protein-Tyrosin-Phosphatase-1B (PTP1B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindung der Formel I in Kombination mit einem HMGCoA-Reduktase Inhibitor, wie Simvastatin, Fluvastatin, Pravastatin, Lovastatin, Atorvastatin, Cerivastatin, Rosuvastatin oder L-659699, verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692), MD-0727 (Microbia Inc., W02005021497) oder mit Verbindungen, wie in WO2002066464 (Kotobuki Pharmaceutical Co. Ltd.), WO2005062824 (Merck & Co.) oder W02005061451 und WO2005061452 (AstraZeneca AB) beschrieben, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, GI 262570, R-483 oder CS-011 (Rivoglitazon), verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101 oder DRF-10945, verabreicht.

Bei einer Ausführungsform der Erfindung wird die Verbindung der Formel I in Kombination mit einem gemischten PPAR alpha/gamma Agonisten, wie z.B. Muraglitazar, Tesaglitazar, Naveglitazar, LY-510929, ONO-5129, E-3030 oder wie in WO00/64888, WO00/64876, WO03/020269, WO2004075891, WO2004076402, WO2004075815, WO2004076447, WO2004076428, WO2004076401, W02004076426, WO2004076427, WO2006018118, WO2006018115, and W02006018116 oder in J.P. Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516, oder wie in WO2005097762, WO2005097786, WO0005097763, WO2006029699 beschrieben verabreicht.

Bei einer Ausführungsform der Erfindung wird die Verbindung der Formel I in Kombination mit Metaglidasen oder mit MBX-2044 oder anderen partiellen PPAR gamma Agonisten/Antagonisten verabreicht.

Bei einer Ausführungsform der Erfindung wird die Verbindung der Formel I in Kombination mit einem Fibrat, wie z.B. Fenofibrat, Clofibrat oder Bezafibrat, verabreicht.

Bei einer Ausführungsform der Erfindung wird die Verbindung der Formel I in Kombination mit einem MTP-Inhibitor, wie z.B. Implitapide, BMS-201038, R-103757 oder solchen wie in WO2005085226 beschrieben, verabreicht.

Bei einer Ausführungsform der Erfindung wird die Verbindung der Formel I in Kombination mit einem CETP-Inhibitor, wie z.B. Torcetrapib oder JTT-705, verabreicht.

Bei einer Ausführungsform der Erfindung wird die Verbindung der Formel I in Kombination mit einem Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin oder Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586 oder solchen wie in W02005097738 beschrieben, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen, verabreicht.

Bei einer Ausführungsform der Erfindung wird die Verbindung der Formel I in Kombination mit einem Vitamin, wie z. B. Vitamin B6 oder Vitamin B12, verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht. Bei einer Ausführungsform der Erfindung wird die Verbindung der Formel I in Kombination mit einem Squalen Synthetase Inhibitor, wie z.B. BMS-188494 oder wie in W02005077907 beschrieben, verabreicht.

Bei einer Ausführungsform der Erfindung wird die Verbindung der Formel I in Kombination mit einem Lipoprotein(a) Antagonist, wie z.B. Gemcabene (CI-1027), verabreicht.

Bei einer Ausführungsform der Erfindung wird die Verbindung der Formel I in Kombination mit einem HM74A Rezeptor Agonisten, wie z.B. Nicotinsäure, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform der Erfindung wird die Verbindung der Formel I in Kombination mit einem Sulfonylharnstoff, wie z.B. Tolbutamid, Glibenclamid, Glipizid oder Glimepirid, verabreicht.

Bei einer Ausführungsform der Erfindung wird die Verbindung der Formel I in Kombination mit einem Biguanid, wie z.B. Metformin, verabreicht.

Bei wieder einer Ausführungsform der Erfindung wird die Verbindung der Formel I in Kombination mit einem Meglitinid, wie z.B. Repaglinide oder Nateglinid, verabreicht.

Bei einer Ausführungsform der Erfindung wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform der Erfindung wird die Verbindung der Formel I in Kombination mit einem α-Glukosidase-Inhibitor, wie z.B. Miglitol oder Acarbose, verabreicht.

Bei einer Ausführungsform der Erfindung wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform der Erfindung wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc., verabreicht.

Bei einer Ausführungsform der Erfindung wird die Verbindung der Formel I in Kombination mit einem Hemmstoff der Glykogen Phosphorylase, wie z.B. PSN-357 oder FR-258900 oder solchen wie in WO2003084922, WO2004007455, WO2005073229-31 oder WO2005067932 beschrieben, verabreicht.

Bei einer Ausführungsform der Erfindung wird die Verbindung der Formel I in Kombination mit Glukagon-Rezeptor-Antagonisten, wie z.B. A-770077, NNC-25-2504 oder wie in WO2004100875 oder WO2005065680 beschrieben, verabreicht.

Bei einer Ausführungsform der Erfindung wird die Verbindung der Formel I in Kombination mit Aktivatoren der Glukokinase, wie z. B. RO-4389620, LY-2121260 (WO2004063179), PSN-105, PSN-110, GKA-50oder solchen wie sie z. B. von Prosidion in WO2004072031, WO2004072066, WO 05103021 oder WO 06016178, von Roche in WO 00058293, WO 00183465, WO 00183478, WO 00185706, WO 00185707, WO 01044216, GB 02385328, WO 02008209, WO 02014312, WO 0246173, WO 0248106, DE 10259786, WO 03095438, US 04067939 oder WO 04052869, von Novo Nordisk in EP 1532980, WO 03055482, WO 04002481, WO 05049019, WO 05066145 oder WO 05123132, von Merck/Banyu in WO 03080585, WO03097824, WO 04081001, WO 05063738 oder WO 05090332, von Eli Lilly in WO 04063194, oder von Astra Zeneca in WO 01020327, WO 03000262, WO 03000267, WO 03015774, WO 04045614, WO 04046139, WO 05044801, WO 05054200, WO 05054233, WO 05056530, WO 05080359, WO 05080360 oder WO 05121110 beschrieben sind, verabreicht.

Bei einer Ausführungsform der Erfindung wird die Verbindung der Formel I in Kombination mit einem Inhibitor der Glukoneogenese, wie z. B. FR-225654, verabreicht.

Bei einer Ausführungsform der Erfindung wird die Verbindung der Formel I in Kombination mit Inhibitoren der Fructose-1,6-bisphosphatase (FBPase), wie z.B. CS-917, verabreicht.

Bei einer Ausführungsform der Erfindung wird die Verbindung der Formel I in Kombination mit Modulatoren des Glukosetransporters-4 (GLUT4), wie z.B. KST-48 (D.-O. Lee et al.: Arzneim.-Forsch. Drug Res. 54 (12), 835 (2004)), verabreicht.

Bei einer Ausführungsform der Erfindung wird die Verbindung der Formel I in Kombination mit Inhibitoren der Glutamin-Fructose-6-Phosphat-Amidotransferase (GFAT), wie sie z. B. in W02004101528 beschrieben sind, verabreicht.

Bei einer Ausführungsform der Erfindung wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X oder wie sie in WO2003074500, WO2003106456, WO200450658, WO2005058901, WO2005012312, WO2005/012308, PCT/EP2005/007821, PCT/EP2005/008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform der Erfindung wird die Verbindung der Formel I in Kombination mit Hemmstoffen der 11-beta-Hydroxysteroid-Dehydrogenase-1 (11ß-HSD1), wie z. B. BVT-2733 oder solche, wie sie z. B. in W0200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004065351, W02004089367, W02004089380, W02004089470-71, WO2004089896, W02005016877 oder W02005097759 beschrieben sind, verabreicht.

Bei einer Ausführungsform der Erfindung wird die Verbindung der Formel I in Kombination mit Inhibitoren der Protein-Tyrosin-Phosphatase-1B (PTP1B), wie sie z. B. in WO200119830-31, WO200117516, WO2004506446, W02005012295, PCT/EP2005/005311, PCT/EP2005/005321, PCT/EP2005/007151, PCT/EP2005/01294 oder DE 10 2004 060542.4 beschrieben sind, verabreicht.

Bei einer Ausführungsform der Erfindung wird die Verbindung der Formel I in Kombination mit Modulatoren des natrium-abhängigen Glukosetransporters 1 oder 2 (SGLT1, SGLT2), wie z.B. KGA-2727, T-1095 und SGL-0010 oder wie sie z. B. in WO2004007517, WO200452903, WO200452902, WO2005121161, WO2005085237, JP2004359630 oder von A. L. Handlon in Expert Opin. Ther. Patents (2005) 15(11), 1531-1540 beschrieben sind, verabreicht.

Bei einer Ausführungsform der Erfindung wird die Verbindung der Formel I in Kombination mit Inhibitoren der hormon-sensitiven Lipase (HSL), wie z. B. in in WO01/17981, WO01/66531, WO2004035550, WO2005073199 oder WO03/051842 beschrieben, verabreicht.

Bei einer Ausführungsform der Erfindung wird die Verbindung der Formel I in Kombination mit Hemmstoffen der Acetyl-CoA Carboxylase (ACC) wie z. B. solchen wie in WO199946262, WO200372197, WO2003072197 oder WO2005044814 beschrieben, verabreicht.

Bei einer Ausführungsform der Erfindung wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform der Erfindung wird die Verbindung der Formel I in Kombination mit einem Inhibitor der Glykogen Synthase Kinase-3 beta (GSK-3 beta), wie z. B. in US2005222220, WO2004046117, WO2005085230, WO2005111018, WO2003078403, WO2004022544, WO2003106410, WO2005058908, US2005038023, WO2005009997, US2005026984, WO2005000836, WO2004106343, EP1460075, WO2004014910, W02003076442, WO2005087727 oder WO2004046117 beschrieben, verabreicht.

Bei einer Ausführungsform der Erfindung wird die Verbindung der Formel I in Kombination mit einem Inhibitor der Protein Kinase C beta (PKC beta), wie z. B. Ruboxistaurin, verabreicht.

Bei einer Ausführungsform der Erfindung wird die Verbindung der Formel I in Kombination mit einem Endothelin-A-Rezeptor Antagonisten, wie z. B. Avosentan (SPP-301), verabreicht.

Bei einer Ausführungsform der Erfindung wird die Verbindung der Formel I in Kombination mit Inhibitoren der "I-kappaB Kinase" (IKK Inhibitoren), wie sie z. B. in W02001000610, WO2001030774, WO2004022553 oder W02005097129 beschrieben sind, verabreicht.

Bei einer Ausführungsform der Erfindung wird die Verbindung der Formel I in Kombination mit Modulatoren des Glukocorticoidrezeptors, wie sie z. B. in W02005090336 beschrieben sind, verabreicht.

Bei einer weiteren Ausführungsform der Erfindung wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558); NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
Peptid YY 3-36 (PYY3-36) oder analoge Verbindungen, wie z. B. CJC-1682 (PYY3-36 konjugiert mit humanem Serum Albumin über Cys34), CJC-1643 (Derivat des PYY3-36, welches sich in vivo an Serum Albumin konjugiert) oder solche, wie sie in WO2005080424 beschrieben sind;
Cannabinoid Rezeptor 1 Antagonisten, wie z.B. Rimonabant, SR147778 oder solche wie sie in z. B. EP 0656354, WO 00/15609, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509 oder WO2005077897 beschrieben sind;
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlorophenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, W02005047251, EP1538159, WO2004072076, WO2004072077 oder WO2006024390 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, W0200185693, W02004085403 oder WO2005075458 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in W0200064884, WO2005082893 beschrieben sind); CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
β3-Agonisten, (wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451));
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71 , GW-803430 oder solche Verbindungen, wie sie in W02003/15769, WO2005085200, W02005019240, WO2004011438, WO2004012648, WO2003015769, WO2004072025, WO2005070898, WO2005070925, WO2006018280, WO2006018279, WO2004039780, WO2003033476, WO2002006245, WO2002002744, WO2003004027 oder FR2868780 beschrieben sind);
CCK-A Agonisten (wie z.B. {2-[4-(4-Chloro-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525), SR-146131 (WO 0244150) oder SSR-125180);
Serotonin-Wiederaufnahme-Inhibitoren (z.B. Dexfenfluramine);
gemischte Serotonin- und noradrenerge Verbindungen (z.B. WO 00/71549); 5-HT-Rezeptor Agonisten z.B. 1-(3-Ethyl-benzofuran-7-yl)-piperazin Oxalsäuresalz (WO 01/09111);
5-HT2C Rezeptor Agonisten (wie z.B. APD-356, BVT-933 oder solche, wie sie in WO200077010, WO20077001-02, W02005019180, WO2003064423, WO200242304 oder WO2005082859 beschrieben sind);
5-HT6 Rezeptor Antagonisten, wie sie z.B. in WO2005058858 beschrieben sind; Bombesin-Rezeptor Agonisten (BRS-3 Agonisten);
Galanin-Rezeptor Antagonisten;
Wachstumshormon (z.B. humanes Wachstumshormon oder AOD-9604); Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01 /85695));
Growth Hormone Secretagogue Receptor Antagonisten (Ghrelin Antagonisten) wie z. B. A-778193 oder solche, wie sie in WO2005030734 beschrieben sind; TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin oder Doprexin);
Lipase/Amylase-Inhibitoren (wie sie z.B. in WO 00/40569 beschrieben sind);
Inhibitoren der Diacylglycerol O-Acyltransferasen (DGATs) wie z. B. in US2004/0224997, WO2004094618, W0200058491, WO2005044250, WO2005072740, JP2005206492 oder WO2005013907 beschrieben;
Inhibitoren der Fettsäuresynthase (FAS) wie z.B. C75 oder solche, wie in WO2004005277 beschrieben;
Oxyntomodulin;
Oleoyl-Estron
oder Agonisten des Schilddrüsenhormonrezeptors (thyroid hormone receptor agonists) wie z. B: KB-2115 oder solche, wie in W020058279, W0200172692, W0200194293, W02003084915, W02004018421 oder W02005092316 beschrieben, verabreicht.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform der Erfindung ist der weitere Wirkstoff Dexamphetamin oder Amphetamin.

Bei einer Ausführungsform der Erfindung ist der weitere Wirkstoff Fenfluramin oder Dexfenfluramin.

Bei noch einer Ausführungsform der Erfindung ist der weitere Wirkstoff Sibutramin.

Bei einer Ausführungsform der Erfindung ist der weitere Wirkstoff Mazindol oder Phentermin.

Bei einer Ausführungsform der Erfindung wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6). Caromax ist ein Carob enthaltendes Produkt der Fa. Nutrinova, Nutrition Specialties & Food Ingredients GmbH, Industriepark Höchst, 65926 Frankfurt / Main) verabreicht. Die Kombination mit Caromax^{®} kann in einer Zubereitung erfolgen oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Bei einer Ausführungsform der Erfindung wird die Verbindung der Formel I in Kombination mit PDE Inhibitoren (Phosphodiesterase), wie sie z. B. in W02003/077949 oder W02005012485 beschrieben sind, verabreicht.

Bei einer Ausführungsform der Erfindung wird die Verbindung der Formel I in Kombination mit NAR-1 (Nicotinic Acid Receptor) Agonisten, wie sie z. B. in W02004094429 beschrieben sind, verabreicht.

Bei einer Ausführungsform der Erfindung wird die Verbindung der Formel I in Kombination mit CB2 (Cannabinoid Receptor) Agonisten, wie sie z. B. in US2005/143448 beschrieben sind, verabreicht.

Bei einer Ausführungsform der Erfindung wird die Verbindung der Formel I in Kombination mit Histamine-1-Agonisten, wie sie z. B. in WO2005101979 beschrieben sind, verabreicht.

Bei einer Ausführungsform der Erfindung wird die Verbindung der Formel I in Kombination mit Bupropion, wie in WO2006017504 beschrieben, verabreicht.

Bei einer Ausführungsform der Erfindung wird die Verbindung der Formel I in Kombination mit Opioid Antagonisten, wie sie z. B. in WO2005107806 oder WO2004094429 beschrieben sind, verabreicht.

Bei einer Ausführungsform der Erfindung wird die Verbindung der Formel I in Kombination mit Neutralen Endopeptidase Inhibitoren , wie sie z. B. in W0200202513, W02002/06492, WO 2002040008, W02002040022 oder W02002047670 beschrieben sind, verabreicht.

Bei einer Ausführungsform der Erfindung wird die Verbindung der Formel I in Kombination mit NPY Inhibitoren (Neuropeptid Y), wie sie z. B. in WO2002047670 beschrieben sind, verabreicht.

Bei einer Ausführungsform der Erfindung wird die Verbindung der Formel I in Kombination mit Natrium/Wasserstoff Austausch Inhibitoren, wie sie z. B. in WO2003092694 beschrieben sind, verabreicht.

Bei einer Ausführungsform der Erfindung wird die Verbindung der Formel I in Kombination mit Modulatoren des Glukocorticoidrezeptors, wie sie z. B. in W02005090336 beschrieben sind, verabreicht.

Bei einer Ausführungsform der Erfindung wird die Verbindung der Formel I in Kombination mit Nikotin Rezeptor Agonisten, wie sie z. B. in W02004094429 beschrieben sind, verabreicht.

Bei einer Ausführungsform der Erfindung wird die Verbindung der Formel I in Kombination mit NRIs (Norepinephrine reuptake inhibitors), wie sie z. B. in W02002053140 beschrieben sind, verabreicht.

Bei einer Ausführungsform der Erfindung wird die Verbindung der Formel I in Kombination mit MOA (E-beta-methoxyacrylate), wie z.B. Segeline oder wie sie z. B. in W02002053140 beschrieben sind, verabreicht.

Bei einer Ausführungsform der Erfindung wird die Verbindung der Formel I in Kombination mit antithrombotischen Wirkstoffen, wie z. B. Clopidrogel, verabreicht. Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

Zu den vorstehend genannten Entwicklungskodes werden nachfolgend teilweise die Formeln aufgeführt.

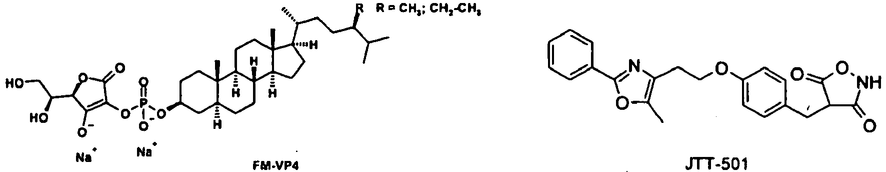

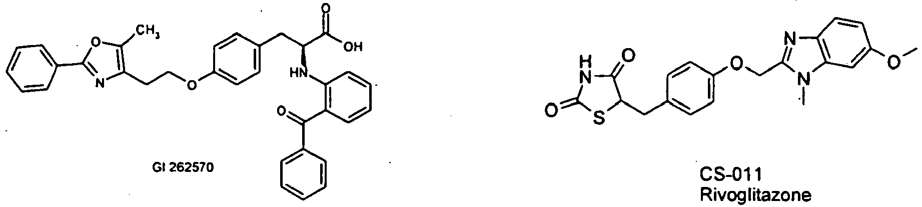

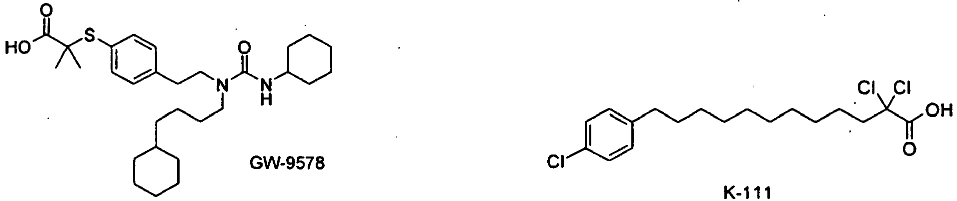

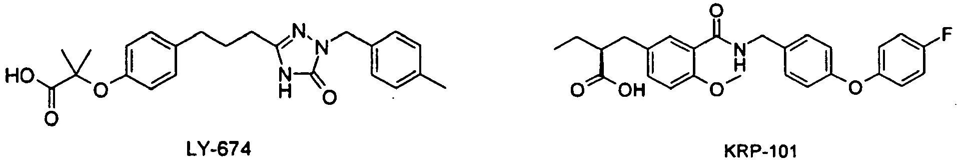

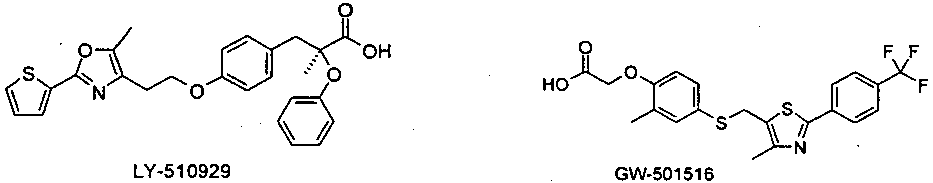

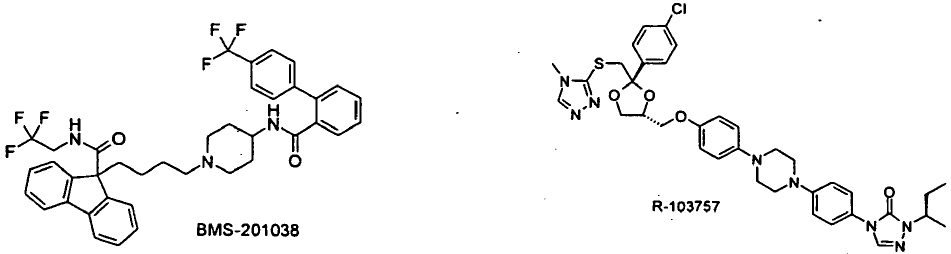

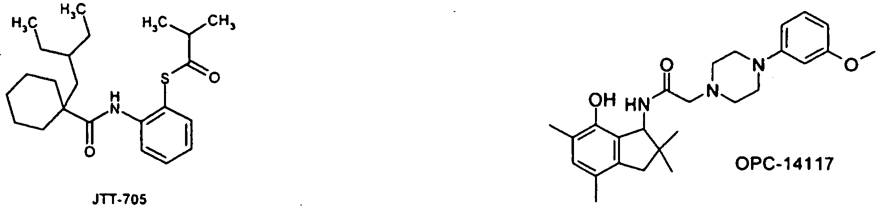

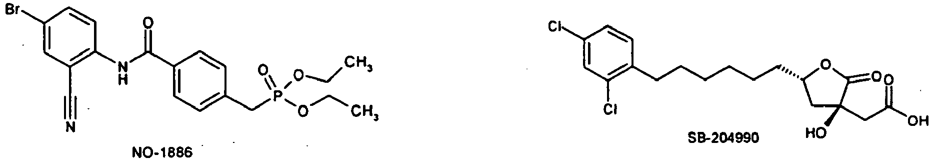

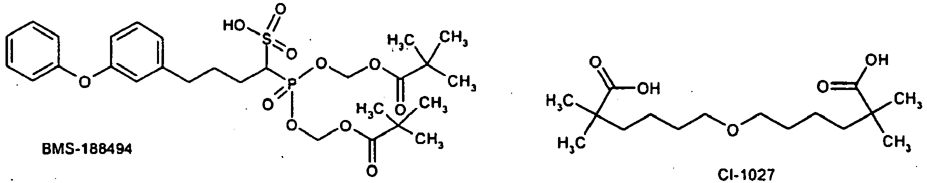

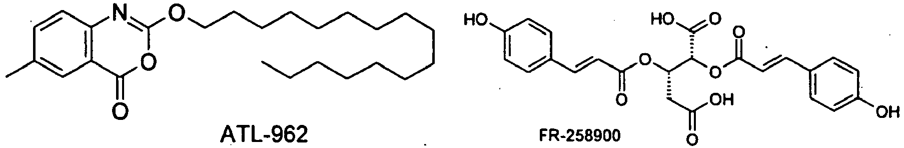

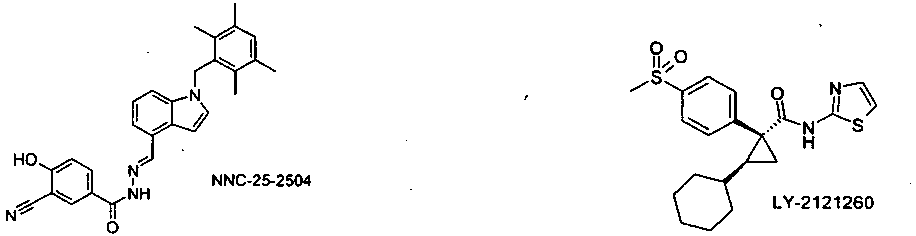

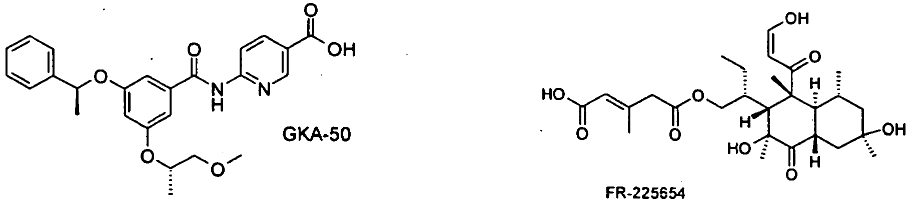

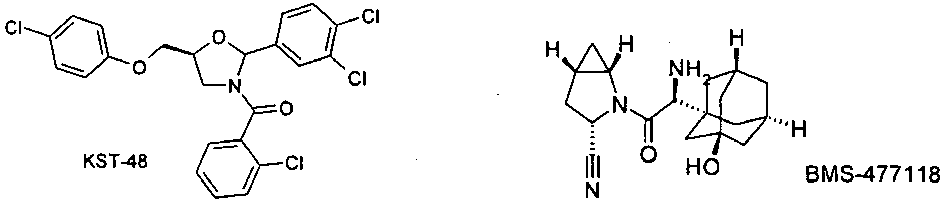

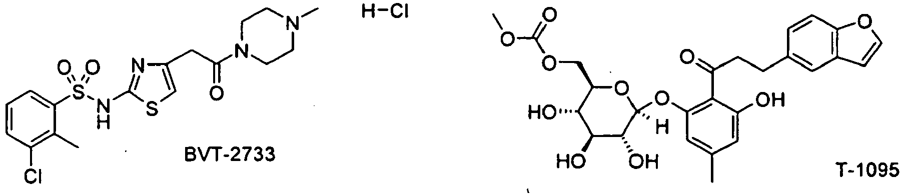

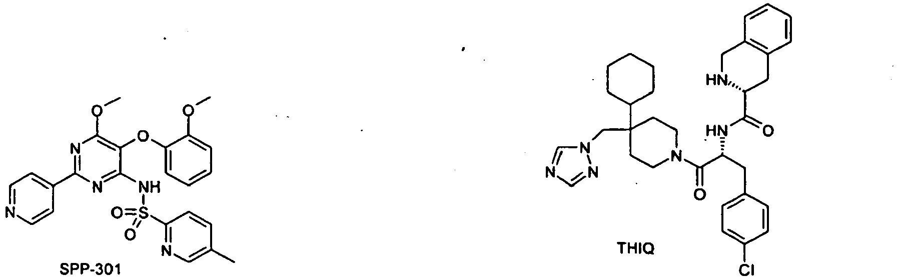

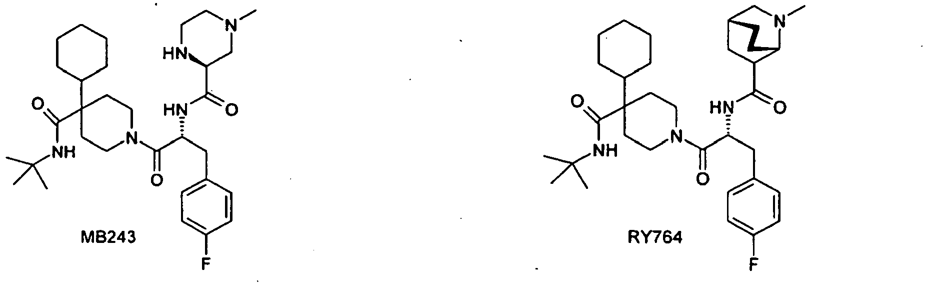

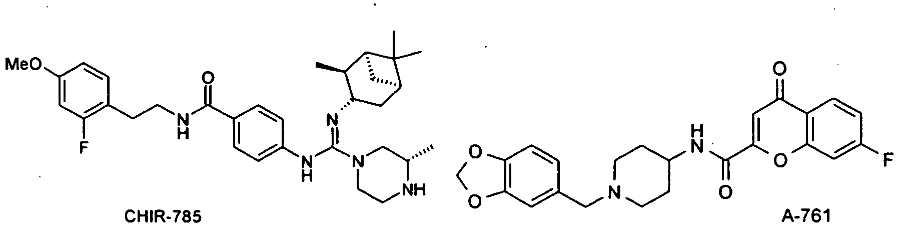

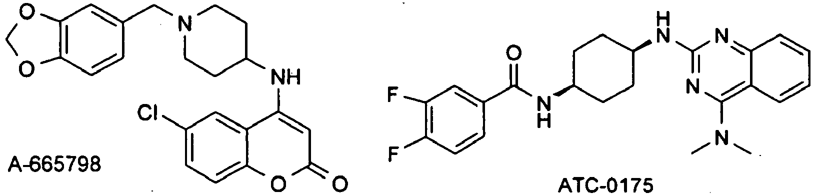

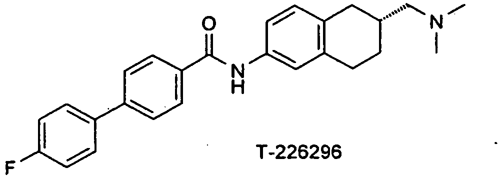

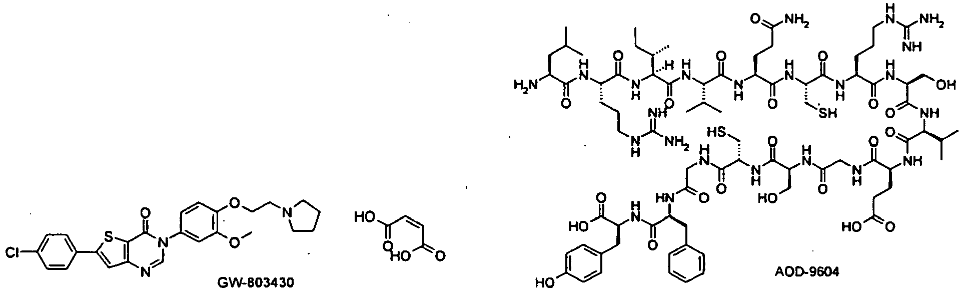

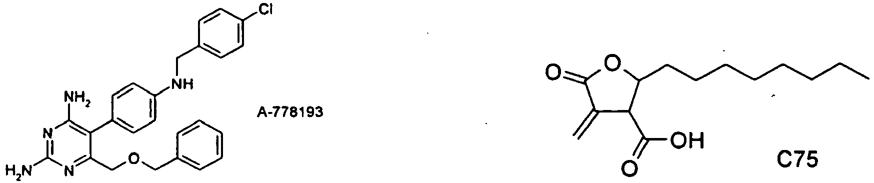

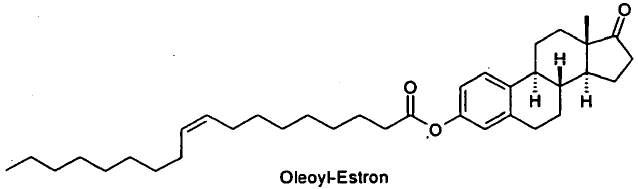

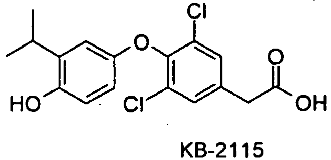

Die Wirksamkeit der erfindungsgemäßen Verbindungen der Formel I wurde an folgenden Enzymtestsystemen geprüft:

### Test auf Hemmung der HSL

### Präparation der partiell gereinigten HSL:

Isolierte Rattenfettzellen werden aus Nebenhodenfettgewebe von nicht-behandelten männlichen Ratten (Wistar, 220-250 g) durch Kollagenasebehandlung gemäß publizierter Verfahren gewonnen (z.B. S. Nilsson et al., Anal. Biochem. 158, 1986, 399 - 407; G. Fredrikson et al., J. Biol. Chem. 256, 1981, 6311 - 6320; H. Tornquist et al., J. Biol. Chem. 251, 1976, 813 - 819). Die Fettzellen aus 10 Ratten werden dreimal durch Flotation mit jeweils 50 ml Homogenisationspuffer (25 mM Tris/HCl, pH 7.4, 0.25 M Sucrose, 1 mM EDTA, 1 mM DTT, 10 µg/ml Leupeptin, 10 µg/ml Antipain, 20 µg/ml Pepstatin) gewaschen und schließlich in 10 ml Homogenisationspuffer aufgenommen. Die Fettzellen werden im Teflon-in-Glas Homogenisator (Braun-Melsungen) durch 10 Hübe bei 1500 rpm und 15°C homogenisiert. Das Homogenisat wird zentrifugiert (Sorvall SM24-Röhrchen, 5000 rpm, 10 min, 4°C). Der Unterstand zwischen der oben liegenden Fettschicht und dem Pellet wird abgenommen und die Zentrifugation wiederholt. Der daraus resultierende Unterstand wird erneut zentrifugiert (Sorvall SM24-Röhrchen, 20000 rpm, 45 min, 4°C). Der Unterstand wird abgenommen und mit 1 g Heparin-Sepharose (Pharmacia-Biotech, CL-6B, 5 x gewaschen mit 25 mM Tris/HCl, pH 7.4, 150 mM NaCl) versetzt. Nach Inkubation für 60 min bei 4°C (in Intervallen von 15 min aufschütteln) wird der Ansatz zentrifugiert (Sorvall SM24-Röhrchen, 3000 rpm, 10 min, 4°C). Der Überstand wird durch Zugabe von Eisessig auf pH 5.2 gebracht und 30 min bei 4°C inkubiert. Die Präzipitate werden durch Zentrifugation (Sorvall SS34, 12000 rpm, 10 min, 4°C) gesammelt und in 2.5 ml 20 mM Tris/HCl, pH 7.0, 1 mM EDTA, 65 mM NaCl, 13 % sucrose, 1 mM DTT, 10 µg/ml Leupeptin/Pepstatin/Antipain suspendiert. Die Suspension wird über Nacht bei 4°C gegen 25 mM Tris/HCl, pH 7.4, 50 % Glyzerin, 1 mM DTT, 10 µg/ml Leupeptin, Pepstatin, Antipain dialysiert und dann auf eine Hydroxiapatit-Säule aufgetragen (0.1 g pro 1 ml Suspension, äquilibriert mit 10 mM Kaliumphosphat, pH 7.0, 30 % Glyzerin, 1 mM DTT). Die Säule wird mit vier Volumina Äquilibrierungspuffer bei einer Flußrate von 20 bis 30 ml/h gewaschen. Die HSL wird mit einem Volumen Äquilibrierungspuffer, der 0.5 M Kaliumphosphat enthält, eluiert, sodann dialysiert (s.o.) und 5- bis 10-fach konzentriert durch Ultrafiltration (Amicon Diaflo PM 10 Filter) bei 4°C. Die partiell gereinigte HSL kann 4 bis 6 Wochen bei -70°C aufbewahrt werden.

### Assay auf HSL-Aktivität:

Für die Herstellung des Substrats werden 25-50 µCi [3H]Trioleoylglycerin (in Toluol), 6.8 µMol unmarkiertes Trioleoylglycerin und 0.6 mg Phospholipide (Phosphatidylcholin/Phosphatidylinositol 3:1 w/v) gemischt, über N2 getrocknet und dann in 2 ml 0.1 M KPi (pH 7.0) durch Ultraschallbehandlung (Branson 250, Mikrospitze, Einstellung 1-2, 2 x 1 min im 1-min Intervall) aufgenommen. Nach Zugabe von 1 ml KPi und erneuter Ultraschallbehandlung (4 x 30 sec auf Eis in 30-sec Intervallen) wird 1 ml 20% BSA (in KPi) hinzugefügt (Endkonzentration Trioleoylglyzerin 1.7 mM). Für die Reaktion werden 100 µl Substratlösung zu 100 µl HSL-Lösung (HSL präpariert wie oben, verdünnt in 20 mM KPi, pH 7.0, 1 mM EDTA, 1 mM DTT, 0.02% BSA, 20 µg/ml Pepstatin, 10 µg/ml Leupeptin) pipettiert und für 30 min bei 37°C inkubiert. Nach Zugabe von 3.25 ml Methanol/Chloroform/Heptan (10:9:7) und von 1.05 ml 0.1 M K2C03, 0.1 M Borsäure (pH 10.5) wird gut gemischt und schließlich zentrifugiert (800 x g, 20 min). Nach Phasentrennung wird ein Äquivalent der oberen Phase (1 ml) abgenommen und die Radioaktivität durch Flüssigszintillationsmessung bestimmt.

Substanzen werden üblicherweise in vier unabhängigen Ansätzen geprüft. Die Hemmung der enzymatischen Aktivität der HSL durch eine Testsubstanz wird durch den Vergleich mit einer nicht-gehemmten Kontrollreaktion bestimmt. Die Berechnung des IC50-Wertes erfolgt über eine Hemmkurve mit mind. 10 Konzentrationen der Testsubstanz. Für die Analyse der Daten wird das Softwarepaket GRAPHIT, Elsevier-BIOSOFT, benutzt.

### Test auf Hemmung der EL:

EL wird als sekretorisches Protein von rekombinanten Zell-Linien (CHO, HEK293) in hoher Konzentration in Zellkulturmedium abgegeben (konditioniertes Medium). Dieses wird nach Aufkonzentration als Enzymlösung eingesetzt.

Zur Charakterisierung der enzymatischen Aktivität von endothelialer Lipase und der Wirkung von Inhibitoren wird das Phospholipase-spezifische Substrat 1,2-bis-(4,4-difluoro-5,7-dimethyl-4-bora-3a,4a-diaza-s-indacene-3-undecanoyl)-sn-glycero-3-phosphocholine, (Hersteller Molecular Probes) verwendet. Durch Hydrolyse der A1 Esterbindung dieses Phospholipids durch das Enzym wird der Fluoreszenzfarbstoff Bodipy freigesetzt, der nach Trennung durch Dünnschichtchromatographie auf einer HPTLC-Platte (Kieselgel 60, Merck) oder direkt im Reaktionsgefäß durch Messen der Fluoreszenz nachgewiesen werden kann. Zur Herstellung der Substratlösung werden 100 µg 1,2-bis-(4,4-difluoro-5,7-dimethyl-4-bora-3a,4a-diaza-s-indacene-3-undecanoyl)-sn-glycero-3-phospho-choline (Hersteller Molecular Probes), 2,4 mg Tripalmitin (Sigma) und 7,9 mg DOP - Cholin (1,2-Dioleoyl-sn-glycero-3-phosphocholin) in 393 µl Chloroform aufgenommen und im Anschluss 157 µl in ein frisches Reaktionsgefäß überführt. Nach Abdampfen des Lösungsmittels wird das Lipidgemisch in 4 ml 200 mM TRIS-HCl, 150 mM Natriumchlorid, pH = 7,4 durch zweimaliges Sonifizieren gelöst. Die anschließende Enzymreaktion erfolgt für 60 Minuten bei 37°C. Hierzu werden 45 µl der Substratlösung mit 1 µl Inhibitor entsprechender Konzentration (gelöst in DMSO, zur Kontrolle wird reine DMSO-Lösung verwendet) und 5 µl Enzymlösung (konditioniertes Medium) inkubiert. Im Anschluß werden 3 µl des Testansatzes auf eine HPTLC-Platte (Kieselgel 60, Merck) aufgetragen und der freigesetzte Fluoreszenzfarbstoff zum Nachweis mit einem Fließmittel (Diäthylether : Petroleumbenzin : Essigsäure [78:22:1]) getrennt. Nach dem Abdampfen des Fließmittels wird die Platte in einen Fluoreszenz-Scanner eingelesen. Als Maß für die Enzymaktivität ist eine gesteigerte Freisetzung des Fluoreszenzfarbstoffes in der ungehemmten Reaktion zu beobachten.

In Abhängigkeit der verwendeten Inhibitorkonzentration ergibt sich eine Reduktion der enzymatischen Aktivität, die Inhibitiorkonzentration, bei welcher eine halbmaximale Enzymaktivität beobachtet wird, wird als IC50 bezeichnet.

In diesen Tests zeigten die Verbindungen der Beispiele folgende von IC₅₀ -Werte:

| Beispiel | IC₅₀ [µM] HSL | IC₅₀ [µM] EL |
|---|---|---|
| 1 | 0,5 | |
| 4 | 0,07 | |
| 5 | 0,3 | |
| 14 | 0,607 | |
| 23 | 0,45 | |
| 24 | 0,175 | |
| 25 | 0,215 | |
| 26 | 0,198 | |
| 30 | 0,585 | |
| 31 | 0,136 | |
| 33 | 0,112 | |
| 35 | 0,78 | |
| 37 | 0,576 | |
| 41 | 0,062 | |
| 42 | 0,159 | |
| 43 | 0,763 | |
| 48 | 0,662 | |
| 50 | 0,367 | |
| 51 | 0,237 | |
| 53 | 0,826 | |
| 54 | | 0,74 |
| 55 | | 0,58 |
| 56 | | 0,04 |
| 61 | 0,03 | |
| 62 | 0,16 | |
| 63 | 0,965 | |
| 64 | 0,03 | |
| 66 | 0,15 | |
| 68 | | 1,24 |

### Verfahren zur Herstellung

Die Herstellung der erfindungsgemäßen Verbindungen der allgemeinen Formel I erfolgt nach an und für sich bekannten Methoden z.B. durch Acylierung von substituierten, bzw. unsubstituierte Triazolopyridinen II mit Carbamoylchloriden III (Methode A), oder in zwei Stufen durch Umsetzung von Triazolopyridinen II mit Phosgen oder Äquivalenten wie Chlorcarbonsäuretrichlormethylester, Carbonsäureditrichlormethylester oder Chlorameisensäure-4-nitrophenylester und weiterer Umsetzung des erhaltenen Triazolopyridin-carbonsäurederivats mit Aminen IV (Methode B). Für Verbindungen bei denen R1 Wasserstoff ist, können die Triazolopyridine II auch mit den entsprechenden Isocyanaten V R1-N=C=O umgesetzt werden.

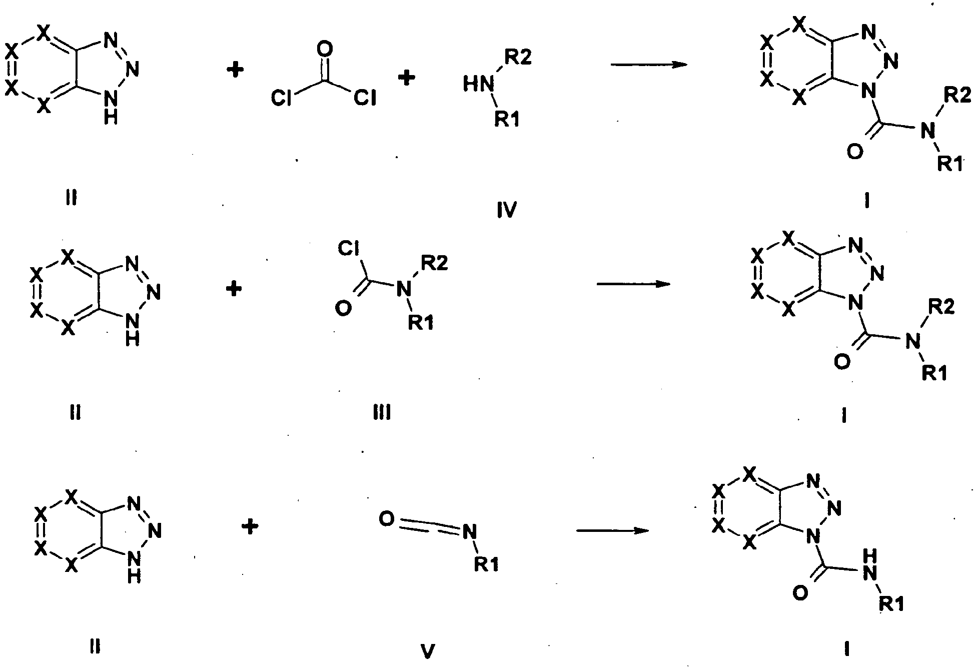

Da bei diesen Reaktionen in der Regel Säuren freigesetzt werden, empfiehlt es sich zur Beschleunigung Basen wie Pyridin, Triethylamin, Natronlauge oder Alkalicarbonate zu zusetzen. Die Reaktionen können in weiten Temperaturbereichen durchgeführt werden. In der Regel hat es sich als vorteilhaft herausgestellt, bei 0°C bis zum Siedepunkt des verwendeten Lösungsmittels zu arbeiten. Als Lösemittel kommen beispielsweise Methylenchlorid, THF, DMF, Toluol, Essigester, n-Heptan, Dioxan, Diethylether oder Pyridin zum Einsatz. Wenn unter wasserfreien Bedingungen gearbeitet wird, haben sich auch starke Basen wie Lithiumhydrid, Natriumhydrid oder Kalium-tert.-butylat in aprotischen Lösungsmitteln wie THF oder DMF bewährt.
Die als Ausgangsverbindungen II eingesetzten Triazolopyridin-derivate sind kommerziell erhältlich oder lassen sich nach Literatur bekannten Verfahren herstellen (z.B. I. Torrini, G. P. Zecchini, F. Agrosi und M. P. Paradisi, J. Het. Chem. (1986) 23, 1459-1463; W. v. Bebenburg, G. Steinmetz, K. Thiele, Chem. Zeit.(1979) 103, 387-399).

Die nachfolgend aufgeführten Beispiele dienen zur Erläuterung der Erfindung, ohne diese jedoch einzuschränken.

### Beispiele

### Allgemeine Arbeitsvorschrift Methode A

Zu einer Lösung von Phosgen (20% in Toluol, 550µL) wird eine Lösung eines sekundären Amins (0,5 mmol) in THF (3 mL) und Pyridin (0,5 mL) bei 25°C zugetropft, und 1 Stunde bei 25°C gerührt. Anschließend wird der Reaktionsansatz im Vakuum zur Trockne eingeengt, der Rückstand in THF (5 mL) aufgenommen und zu einer Lösung von 1H-[1,2,3]triazolo[4,5-b]pyridin (60 mg, 0,5 mmol) in Pyridin (5mL) gegeben. Der Ansatz wird 16 h bei 25°C gerührt und anschließend im Vakuum zur Trockne eingeengt.

Das Produkt wird als Gemisch der Regioisomeren erhalten, die durch bekannte Methoden, insbesondere chromatographische Methoden getrennt werden können.

### 4-Methyl-piperidin-1-carbonyl chlorid

9 g (90.75 mmol) 4-Methyl-piperidin und 13.9 ml (100 mmol) Triethylamin wurden bei-30°C zu 54.9 ml (100 mmol) Phosgen in Toluol (20 proz.) und 100 ml THF zugegeben, 2.5 h gerührt und dann das Reaktionsgemisch auf Raumtemperatur erwärmen lassen. Das Reaktionsgemisch wurde eingeengt und der Rückstand mit Methylenchlorid versetzt, abfiltriert und das Filtrat eingeengt. Das Rohprodukt (12.7 g) wurde ohne weitere Reinigung umgesetzt.

### Beispiel 5 (Methode A):

(6-Chlor-[1,2,3]triazolo[4,5-b]pyridin-1-yl)-(4-methyl-piperidin-1-yl)- methanon

6-Chlor-1 H-[1,2,3]triazolo[4,5-b]pyridin (200 mg, 1.29 mmol) und 4-Methyl-piperidin-1-carbonyl chlorid (418 mg, 2.56 mmol) wurden in 10 ml Pyridin gelöst. Dann wurde Triethylamin (180 µl, 3.88 mmol) zugegeben und 4 h bei Raumtemperatur gerührt und über Nacht stehen lassen. Das Reaktionsgemisch wurde eingeengt und der Rückstand mit Wasser und Essigsäureethylester versetzt. Die organische Phase wurde abgetrennt, die Wasserphase nochmals extrahiert und die vereinigten organischen Phasen mit Wasser gewaschen und eingeengt. Das Rohprodukt wurde über präparative HPLC (PR18, Acetonitril/Wasser 0.1% TFA) gereinigt. Ausbeute: 106 mg (29%) (6-Chlor-[1,2,3]triazolo[4,5-b]pyridin-1-yl)-(4-methyl-piperidin-1-yl)- methanon , M+H+: 280.2.

### Beispiel 3 und 66:

(5-Chlor-[1,2,3]triazolo[4,5-b]pyridin-1-yl)-(3,4-dihydro-1H-isochinolin-2-yl)- methanon und
(5-Chlor-[1,2,3]triazolo[4,5-b]pyridin-3-yl)-(3,4-dihydro-1H-isochinolin-2-yl)- methanon 5-Chlor-3H-[1,2,3]triazolo[4,5-b]pyridin (500 mg, 3.23 mmol) und 3,4-Dihydro-1H-isochinolin-2-carbonyl chlorid (950 mg, 4.85 mmol) wurden in 25 ml Pyridin gelöst. Dann wurde Triethylamin (675 µl, 4.85 mmol) zugegeben und 9 h bei Raumtemperatur gerührt und über Nacht stehen lassen. Das Reaktionsgemisch wurde eingeengt und der Rückstand mit Wasser und Essigsäureethylester versetzt. Die organische Phase wurde abgetrennt, die Wasserphase nochmals extrahiert und die vereinigten organischen Phasen mit Wasser gewaschen und eingeengt. Das Rohprodukt wurde über präparative HPLC (PR18, Acetonitril/Wasser 0.1 % TFA) gereinigt. Ausbeute: 183 mg (5-Chlor-[1,2,3]triazolo[4,5-b]pyridin-1-yl)-(3,4-dihydro-1H-isochinolin-2-yl)-methanon, M+H+: 314.03 und 23 mg (5-Chlor-[1,2,3]triazolo[4,5-b]pyridin-3-yl)-(3,4-dihydro-1H-isochinolin-2-yl)-methanon, M+H+: 314.03.

Beispiel 1 und 61:

(5-Chlor-[1,2,3]triazolo[4,5-b]pyridin-1-yl)-(4-methyl-piperidin-1-yl)- methanon und (5-Chlor-[1,2,3]triazolo[4,5-b]pyridin-3-yl)-(4-methyl-piperidin-1-yl)- methanon

5-Chlor-3H-[1,2,3]triazolo[4,5-b]pyridin (100 mg, 0.65 mmol) wurden in 10 ml THF gelöst. Dann wurden Triethylamin (135 µl, 0.97 mmol) und 4-Methyl-piperidin-1-carbonyl chlorid (146 mg, 0.9 mmol) zugegeben und 4 h bei Raumtemperatur gerührt und über Nacht stehengelassen. Das Reaktionsgemisch wurde eingeengt und der Rückstand mit Wasser und Essigsäureethylester versetzt. Die organische Phase wurde abgetrennt, die Wasserphase nochmals extrahiert und die vereinigten organischen Phasen mit Wasser gewaschen und eingeengt. Das Rohprodukt wurde über präparative HPLC (PR18, Acetonitril/Wasser 0.1 % TFA) gereinigt. Ausbeute: 25 mg (14%)(5-Chlor-[1,2,3]triazolo[4,5-b]pyridin-3-yl)-(4-methyl-piperidin-1-yl)-methanon, M+H+: 280.04 und 41 mg (23%)(5-Chlor-[1,2,3]triazolo[4,5-b]pyridin-1-yl)-(4-methyl-piperidin-1-yl)-methanon M+H+: 280.04.

### Beispiel 57 und 59:

(4-Methyl-piperidin-1-yl)-[1,2,3]triazolo[4,5-c]pyridin-1-yl-methanon und (4-Methyl-piperidin-1-yl)-[1,2,3]triazolo[4,5-c]pyridin-3-yl-methanon

3H-[1,2,3]Triazolo[4,5-c]pyridin (50 mg, 0.41 mmol) wurden in 10 ml Pyridin gelöst. Dann wurden Triethylamin (135 µl, 0.97 mmol) und 4-Methyl-piperidin-1-carbonyl chlorid (101 mg, 0.62 mmol) zugegeben und 5 h bei 120°C gerührt und über Nacht stehengelassen. Das Reaktionsgemisch wurde eingeengt und der Rückstand mit Wasser und Essigsäureethylester versetzt. Die organische Phase wurde abgetrennt, die Wasserphase nochmals extrahiert und die vereinigten organischen Phasen mit Wasser gewaschen und eingeengt. Das Rohprodukt wurde über präparative HPLC (PR18, Acetonitril/Wasser 0.1 % TFA) gereinigt. Ausbeute:
13 mg (13%) (4-Methyl-piperidin-1-yl)-[1,2,3]triazolo[4,5-c]pyridin-1-yl-methanon, M+H+: 246.15 und
34 mg (33%) (4-Methyl-piperidin-1-yl)-[1,2,3]triazolo[4,5-c]pyridin-3-yl-methanon M+H+: 246.15.

### Analog wurden die folgenden Beispiele hergestellt:

### (Die Identität der Verbindungen wurde durch Massenspektrometrie geprüft)

| Beispiel | Struktur |
|---|---|
| 1 | 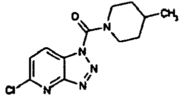 |
| 2 | 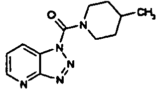 |
| 3 | 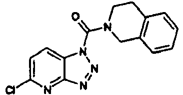 |
| 4 | 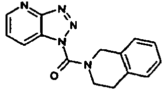 |
| 5 | 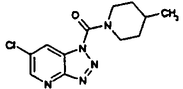 |
| 6 | 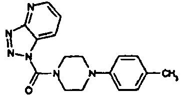 |
| 7 | 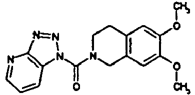 |
| 8 | 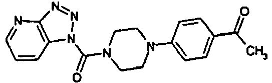 |
| 9 | 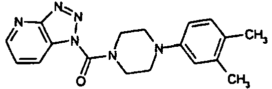 |
| 10 | 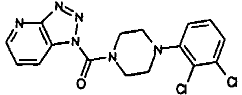 |
| 11 | 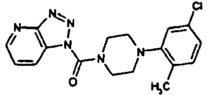 |
| 12 | 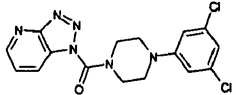 |
| 13 | 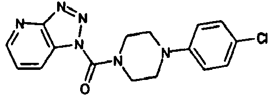 |
| 14 | 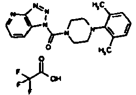 |
| 15 | 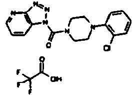 |
| 16 | 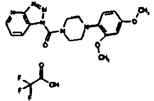 |
| 17 | 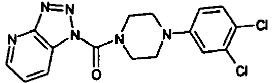 |
| 18 | 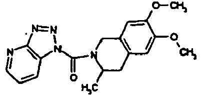 |
| 19 | 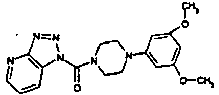 |
| 20 | 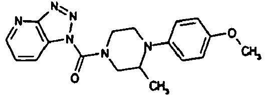 |
| 21 | 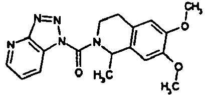 |
| 22 | 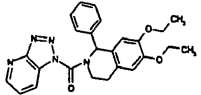 |
| 23 | 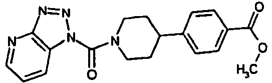 |
| 24 | 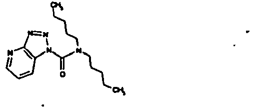 |
| 25 | 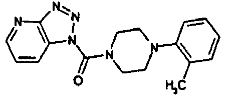 |
| 26 | 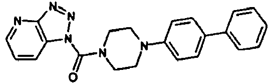 |
| 27 | 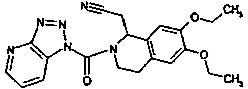 |
| 28 | 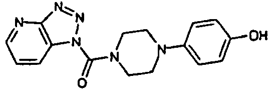 |
| 29 | 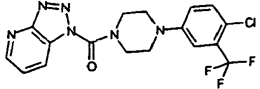 |
| 30 | 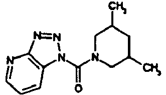 |
| 31 | 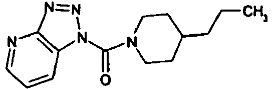 |
| 32 | 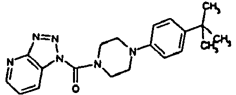 |
| 33 | 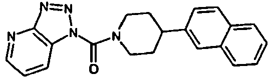 |
| 34 | 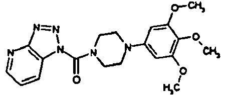 |
| 35 | 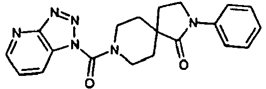 |
| 36 | 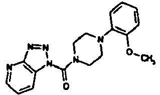 |
| 37 | 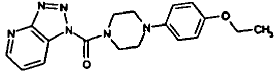 |
| 38 | 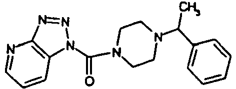 |
| 39 | 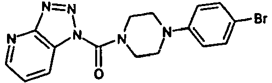 |
| 40 | 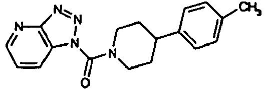 |
| 41 | 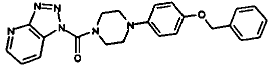 |
| 42 | 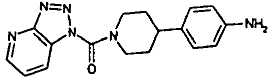 |
| 43 | 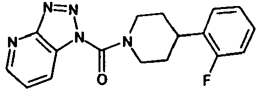 |
| 44 | 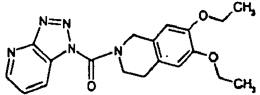 |
| 45 | 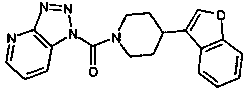 |
| 46 | 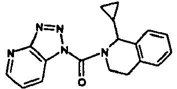 |
| 47 | 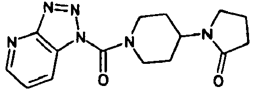 |
| 48 | 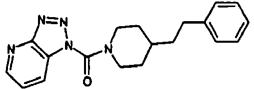 |
| 49 | 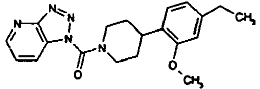 |
| 50 | 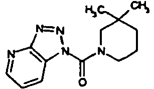 |
| 51 | 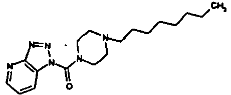 |
| 52 | 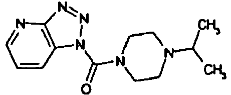 |
| 53 | 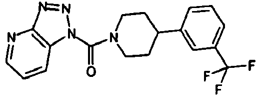 |
| 54 | 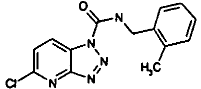 |
| 55 | 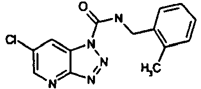 |
| 56 | 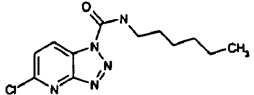 |
| 57 | 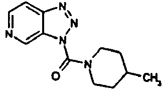 |
| 58 | 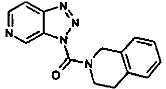 |
| 59 | 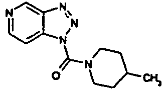 |
| 60 | 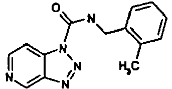 |
| 61 | 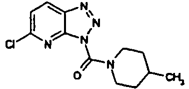 |
| 62 | 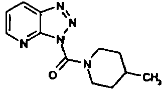 |
| 63 | 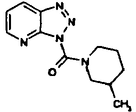 |
| 64 | 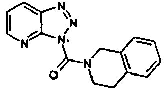 |
| 65 | 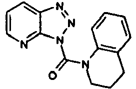 |
| 66 | 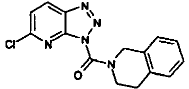 |
| 67 | 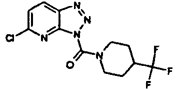 |
| 68 | 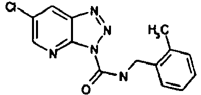 |
| 69 | 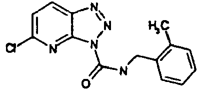 |

## Patentansprüche

1. Verbindung der allgemeinen Formel I 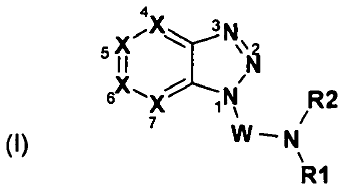 wobei bedeuten:
X gleich oder verschieden =C(-R)- oder =N-, wobei ein X für =N- steht;
W -(C=O)-, -(S=O)-, -(SO₂)-;
R gleich oder verschieden Wasserstoff, Halogen, (C₁-C₆)-Alkyl, (C₁-C₃)-Haloalkyl, (C₁-C₃)-Alkyloxy-(C₁-C₃)-Alkylen, Hydroxy, Phenoxy, Amino, (C₁-C₆)-Alkylamino, di-(C₂-C₁₂)-Alkylamino, Cyano, Nitro, COOR3, mono-(C₁-C₆)-Alkylaminocarbonyl, di-(C₂-C₈)-Alkylaminocarbonyl, (C₁-C₆)-Alkylmercapto, (C₁-C₆)-Alkylsulfinyl, (C₁-C₆)-Alkylsulfonyl, Aminosulfonyl, Pentafluorsulfanyl, Aryl, Heteroaryl, (C₃-C₁₂)-Cycloalkyl, (C₁-C₆)-Alkylcarbonyl, O-CO-NR3R3a, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR3R3a oder unsubstituiertes oder ein oder mehrfach durch F substituiertes (C₁-C₆)-Alkyloxy;
R1 (C₅-C₁₆)-Alkyl, (C₅-C₁₂)-Cycloalkyl, Y-Aryl, Y-Heteroaryl, Y-(C₅-C₁₂)-Cycloalkyl, Bicyclus, wobei Aryl, Heteroaryl, Cycloalkyl oder Bicyclus durch Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkyloxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein oder mehrfach substituiert sein kann;
Y (C₁-C₃)-Alkylen, welches durch Halogen, (C₁-C₂)-Alkyl oder Hydroxy ein- oder mehrfach substituiert sein kann;
R2 Wasserstoff, (C₄-C₁₂)-Alkyl; oder
R1 und R2 zusammen mit dem sie tragenden Stickstoffatom ein monocyclisches, gesättigtes oder teilweise ungesättigtes 4- bis 7-gliedriges Ringsystem oder ein bicyclisches gesättigtes oder teilweise ungesättigtes 8- bis 14 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, -SO-, - SO₂-, ersetzt sein können, mit der Maßgabe, dass zwei Einheiten aus der Reihe -O-, -S-, -SO-, -SO₂- nicht benachbart sein dürfen;
R3, R3a gleich oder verschieden Wasserstoff, (C₁-C₆)-Alkyl, Benzyl;
R4, R5 gleich oder verschieden (C₁-C₁₂)-Alkyl, (C₁-C₆)-Alkyloxy, Halogen, (C₁-C₃)-Haloalkyl, (C₁-C₃)-Haloalkyloxy, COOR3, Nitro, Cyano, Aryl, Heterocyclus, (C₃-C₁₂)-Cycloalkyl, (C₁-C₃)-Alkylen-Aryl, (C₁-C₃)-Alkylen-Heterocyclus, (C₁-C₃)-Alkylen-Cycloalkyl, wobei Aryl, Heterocyclus oder Cycloalkyl ein oder mehrfach durch Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, NR3R3a substituiert sein kann;
die tautomeren Formen der Verbindungen sowie deren physiologisch verträgliche Salze,
mit der Maßgabe, dass die Verbindung mit den Bedeutungen X in 4, 5 und 6-Position =C(-R)-, in 7-Position =N-, W ist -(C=O)-, R ist H und R1 und R2 bilden ein Morpholin ausgenommen ist.

2. Verbindung der Formel I gemäß Anspruch 1, **dadurch gekennzeichnet, dass**
X gleich oder verschieden =C(-R)- oder =N-, wobei ein X für =N- steht;
W -(C=O)-;
bedeuten.

3. Verbindung der Formel I gemäß Anspruch 1, **dadurch gekennzeichnet, dass**
X gleich oder verschieden =C(-R)- oder =N-, wobei ein X für =N- steht;
W -(C=O)-;
R gleich oder verschieden Wasserstoff, Halogen, (C₁-C₆)-Alkyl, Trifluormethyl, (C₁-C₃)-Alkyloxy-(C₁-C₃)-Alkylen, Hydroxy, Phenoxy, Amino, (C₁-C₆)-Alkylamino, di-(C₂-C₁₂)-Alkylamino, Cyano, Nitro, COOR3, mono-(C₁-C₆)-Alkylaminocarbonyl, di-(C₂-C₈)-Alkylaminocarbonyl, (C₁-C₆)-Alkylsulfonyl, Aminosulfonyl, Pentafluorsulfanyl, Phenyl, (C₁-C₆)-Alkylcarbonyl, O-CO-NR3R3a, O-CO-(C₁-C₆)-Alkylen-CO-O-(C₁-C₆)-Alkyl, O-CO-(C₁-C₆)-Alkylen-CO-OH, O-CO-(C₁-C₆)-Alkylen-CO-NR3R3a oder unsubstituiertes oder ein oder mehrfach durch F substituiertes (C₁-C₆)-Alkyloxy;
R1 (C₆-C₁₂)-Alkyl, (C₅-C₁₂)-Cycloalkyl, Y-Phenyl, Y-Heteroaryl, Bicyclus, wobei Phenyl, Heteroaryl, Cycloalkyl oder Bicyclus durch Halogen, (C₁-C₆)-Alkyl, (C₁-C₆)-Alkyloxy, Hydroxy, (C₁-C₆)-Alkylmercapto, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkyloxycarbonyl, (C₁-C₆)-Alkylcarbonyl, Cyano, Nitro, Trifluormethyl, Trifluormethyloxy, (C₁-C₆)-Alkylsulfonyl, Aminosulfonyl ein oder mehrfach substituiert sein kann;
Y -CH₂-, welches durch Fluor, Methyl oder Hydroxy einfach substituiert sein kann;
R2 Wasserstoff, (C₅-C₁₀)-Alkyl; oder
R1 und R2 zusammen mit dem sie tragenden Stickstoffatom ein monocyclisches, gesättigtes oder teilweise ungesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, -SO₂-, ersetzt sein können, mit der Maßgabe, dass zwei Einheiten aus der Reihe -O-, -S-, -SO₂- nicht benachbart sein dürfen;
R3, R3a gleich oder verschieden Wasserstoff, (C₁-C₆)-Alkyl, Benzyl;
R4, R5 gleich oder verschieden (C₁-C₁₀)-Alkyl, (C₁-C₆)-Alkyloxy, Halogen, (C₁-C₃)-Haloalkyl, (C₁-C₃)-Haloalkyloxy, COOR3, Nitro, Cyano, Aryl, Heterocyclus, (C₃-C₇)-Cycloalkyl, (C₁-C₃)-Alkylen-Aryl, (C₁-C₃)-Alkylen-Heteroaryl, wobei Aryl, Heterocyclus oder Cycloalkyl ein oder mehrfach durch Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, NR3R3a substituiert sein kann;
bedeuten,
die tautomeren Formen der Verbindungen sowie deren physiologisch verträgliche Salze.

4. Verbindung der Formel I gemäß Ansprüchen 1 oder 3, **dadurch gekennzeichnet, dass**
X gleich oder verschieden =C(-R)- oder =N-, wobei ein X für =N- steht;
W -(C=O)-;
R gleich oder verschieden Wasserstoff, Hälogen, (C₁-C₆)-Alkyl, Trifluormethyl, Hydroxy, Amino, Cyano, COOR3, mono-(C₁-C₆)-Alkylaminocarbonyl, di-(C₂-C₈)-Alkylaminocarbonyl, (C₁-C₆)-Alkylsulfonyl, Aminosulfonyl, Pentafluorsulfanyl, (C₁-C₆)-Alkylcarbonyl, O-CO-NR3R3a, O-CO-(C₁-C₆)-Alkylen-CO-O-(C₁-C₆)-Alkyl oder unsubstituiertes oder ein oder mehrfach durch F substituiertes (C₁-C₆)-Alkyloxy;
R1 (C₆-C₁₂)-Alkyl, Y-Phenyl, Y-Heteroaryl, Bicyclus, wobei Aryl, Heteroaryl oder Bicyclus durch Halogen, (C₁-C₆)-Alkyl, (C₁-C₆)-Alkyloxy, Hydroxy, Amino, (C₁-C₆)-Alkylamino, di-(C₂-C₁₂)-Alkylamino, mono-(C₁-C₆)-Alkylaminocarbonyl, di-(C₂-C₈)-Alkylaminocarbonyl, (C₁-C₆)-Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein oder mehrfach substituiert sein kann;
Y -CH₂-;
R2 Wasserstoff, (C₅-C₁₀)-Alkyl; oder
R1 und R2 zusammen mit dem sie tragenden Stickstoffatom ein monocyclisches, gesättigtes oder teilweise ungesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O- -S-, ersetzt sein können, mit der Maßgabe, dass zwei Einheiten aus der Reihe -O-, -S-, nicht benachbart sein dürfen;
R3, R3a gleich oder verschieden Wasserstoff, (C₁-C₆)-Alkyl, Benzyl;
R4, R5 gleich oder verschieden (C₁-C₁₀)-Alkyl, (C₁-C₆)-Alkyloxy, Halogen, (C₁-C₃)-Haloalkyl, (C₁-C₃)-Haloalkloxy, COOR3, Nitro, Cyano, Aryl, Heteroaryl, (C₃-C₇)-Cycloalkyl, (C₁-C₃)-Alkylen-Aryl, (C₁-C₃)-Alkylen-Heteroaryl, wobei Aryl, Heteroaryl oder Cycloalkyl ein oder mehrfach durch Halogen, (C₁-C₆)-Alkyl, (C₁-C₆)-Alkyloxy, O-(C₁-C₃)-Alkylen-Aryl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, Amino substituiert sein kann;
bedeuten,
die tautomeren Formen der Verbindungen sowie deren physiologisch verträgliche Salze.

5. Verbindung der Formel I gemäß Ansprüchen 1 bis 4, **dadurch gekennzeichnet, dass**
X gleich oder verschieden =C(-R)- oder =N-, wobei ein X für =N- steht;
W -(C=O)-;
R gleich oder verschieden Wasserstoff, Halogen, (C₁-C₆)-Alkyl, Trifluormethyl, Hydroxy, Amino, Cyano, COOR3, (C₁-C₆)-Alkylsulfonyl, Aminosulfonyl, (C₁-C₆)-Alkylcarbonyl oder unsubstituiertes oder ein oder mehrfach durch F substituiertes (C₁-C₆)-Alkyloxy;
R1 (C₆-C₁₂)-Alkyl, Y-Phenyl, wobei Phenyl durch Halogen, (C₁-C₆)-Alkyl, (C₁-C₆)-Alkyloxy, Hydroxy, Amino, (C₁-C₆)-Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein bis dreifach substituiert sein kann;
Y -CH₂-;
R2 Wasserstoff, (C₅-C₁₀)-Alkyl; oder
R1 und R2 zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden können, deren einzelne Glieder der Ringsysteme durch ein bis drei Atome oder Atomgruppen aus der Reihe -CHR4-, -CR4R5-, =(C-R4)-, -NR5-, -C(=O)-, -O-, -S-, ersetzt sein können, mit der Maßgabe, dass zwei Einheiten aus der Reihe -O-, -S-, nicht benachbart sein dürfen;
R3, R3a gleich oder verschieden Wasserstoff, (C₁-C₆)-Alkyl;
R4, R5 gleich oder verschieden (C₁-C₁₀)-Alkyl, (C₁-C₆)-Alkyloxy, Halogen, Trifluormethyl, Trifluormethoxy, COOR3, Cyano, Aryl, Cyclopropyl, (C₁-C₃)-Alkylen-Phenyl, wobei Aryl ein oder mehrfach durch Halogen, (C₁-C₆)-Alkyl, (C₁-C₆)-Alkyloxy, O-(C₁-C₃)-Alkylen-Phenyl, Trifluormethyl, Trifluormethoxy, Hydroxy, mono-(C₁-C₆)-Alkylaminocarbonyl, di-(C₂-C₈)-Alkylaminocarbonyl, CO-(C₁-C₆)-Alkyl, CO-O-(C₁-C₆)-Alkyl, Amino substituiert sein kann;
bedeuten,
die tautomeren Formen der Verbindungen sowie deren physiologisch verträgliche Salze.

6. Verbindung der Formel I gemäß Ansprüchen 1 bis 5, **dadurch gekennzeichnet, dass**
NR1R2 für ein monocyclisches gesättigtes 5- bis 6-gliedriges Ringsystem steht, das in 4 Position ein Atom oder Atomglied aus der Reihe -CHR4-, - CR4R5-, -NR5-, -(C=O) enthält oder für ein bicyclisches gesättigtes oder teilweise ungesättigtes 9- bis 10 gliedriges Ringsystem, dessen einzelne Glieder des Ringsystems durch ein oder zwei Atomgruppen aus der Reihe -CHR4-, -(C=R4)-, =(C-R4)-, -NR5-, -(C=O)- ersetzt sein können.

7. Verbindung der Formel I gemäß Ansprüchen 1 bis 6, **dadurch gekennzeichnet, dass**
R1 und R2 zusammen mit dem sie tragenden Stickstoffatom ein monocyclisches, gesättigtes 5- bis 6-gliedriges Ringsystem oder ein bicyclisches gesättigtes oder teilweise ungesättigtes 9- bis 10 gliedriges Ringsystem bilden, deren einzelne Glieder der Ringsysteme durch eine Atomgruppe aus der Reihe -CHR4-, -NR5 ersetzt sein können.

8. Verbindung der Formel I gemäß Ansprüchen 1 bis 7, **dadurch gekennzeichnet, dass**
X gleich oder verschieden =C(-R)- oder =N-, wobei ein X für =N- steht;
R gleich oder verschieden Wasserstoff, Cl;
NR1R2 für Piperidin steht, das in 4 Position das Atomglied -CHR4- enthält; oder für Piperazin steht, das in 4-Position das Atomglied -NR5 enthält;
R4, R5 (C₁-C₈)-Alkyl, Phenyl, -(C₁-C₂)-Alkylen-Phenyl, Naphtyl, Pyrrolidin-2-on, Benzofuranyl, wobei Phenyl ein-, zwei- oder dreifach durch Methyl, Ethyl, t-Butyl, Methoxy, Ethoxy, F, Cl, Br, OH, CF₃, NH₂, CO-OCH₃, CO-CH₃, Phenyl, -O-Benzyl substituiert sein kann;
bedeuten.

9. Verbindung der Formel gemäß Ansprüchen 1 bis 8, **dadurch gekennzeichnet, dass**
NR1R2 für Piperidin steht, das in 4 Position das Atomglied -CHR4- enthält oder für Piperazin steht, das in 4-Position das Atomglied -NR5 enthält;
R4, R5 Methyl oder Phenyl, welches durch Methyl, Methoxy, Ethoxy, F, Cl, Br, OH, CF₃, NH₂, CO-OCH₃, CO-CH₃, Phenyl, -O-Benzyl ein oder zweifach substituiert sein kann; bedeutet.

10. Verbindung der Formel gemäß Ansprüchen 1 bis 5, **dadurch gekennzeichnet, dass**
X gleich oder verschieden =C(-R)- oder =N-, wobei ein X für =N- steht;
R gleich oder verschieden Wasserstoff, Cl; bedeutet;
NR1R2 für Tetrahydrochinolin oder Tetrahydroisochinolin steht, welches durch Methoxy ein oder zweifach substituiert sein kann.

11. Verbindung der Formel I gemäß Ansprüchen 1 bis 5, **dadurch gekennzeichnet, dass**
R2 Wasserstoff bedeutet.

12. Verbindung der Formel I gemäß Ansprüchen 1 bis 5, **dadurch gekennzeichnet, dass**
R1 (C₆-C₁₂)-Alkyl, Y-Phenyl, wobei Phenyl durch Halogen, (C₁-C₆)-Alkyl, (C₁-C₆)-Alkyloxy, Hydroxy, Amino, (C₁-C₆)-Alkyloxycarbonyl, (C₁-C₆)-Alkylcarbonyl, Cyano, Trifluormethyl, Trifluormethyloxy, (C₁-C₆)-Alkylsulfonyl, Aminosulfonyl ein bis dreifach substituiert sein kann;
Y -CH₂-;
R2 Wasserstoff;
bedeuten.

13. Verbindung der Formel I gemäß Ansprüchen 1 bis 5, **dadurch gekennzeichnet, dass**
X gleich oder verschieden =C(-R)- oder =N-, wobei ein X für =N- steht;
R gleich oder verschieden Wasserstoff, Cl;
R1 Hexyl, Y-Phenyl, wobei Phenyl durch Methyl, bevorzugt in 2-Position, substituiert sein kann;
Y -CH₂-;
R2 Wasserstoff;
bedeuten.

14. Verbindung der Formel I gemäß Ansprüchen 1 bis 13, **dadurch gekennzeichnet, dass**
X in Position 4, 5 und 6 für gleich oder verschieden =C(-R)-, in Position 7 für =N- steht.

15. Verbindung der Formel 1, gemäß Ansprüchen 1 bis 13, **dadurch gekennzeichnet, dass**
X gegebenenfalls in Position 4 oder 7 für =C(-R)- mit R = Wasserstoff oder =N- steht, in Position 5 oder 6 für =C(-R)- mit R nicht Wasserstoff steht.

16. Arzneimittel enthaltend eine oder mehrere Verbindungen der Formel I gemäß den Ansprüchen 1 bis 15.

17. Verwendung der Verbindungen der Formel I gemäß den Ansprüchen 1 bis 15 zur Herstellung eines Arzneimittels zur Behandlung und/oder Prävention von Störungen des Fettsäurestoffwechsels und Glucoseverwertungsstörungen.

18. Verwendung der Verbindungen der Formel I gemäß den Ansprüchen 1 bis 15 zur Herstellung eines Arzneimittels zur Behandlung und/oder Prävention von Störungen, bei denen Insulin Resistenz eine Rolle spielt.

19. Verwendung der Verbindungen der Formel I gemäß den Ansprüchen 1 bis 15 zur Herstellung eines Arzneimittels zur Behandlung und/oder Prävention von Diabetes mellitus und der damit verbundenen Folgeerkrankungen.

20. Verwendung der Verbindungen der Formel gemäß den Ansprüchen 1 bis 15 zur Herstellung eines Arzneimittels zur Behandlung und/oder Prävention von Dyslipidämien und deren Folgen.

21. Verwendung der Verbindungen der Formel I gemäß den Ansprüchen 1 bis 15 zur Herstellung eines Arzneimittels zur Behandlung und/oder Prävention von Zuständen, die mit dem Metabolischen Syndrom assoziert sind.

22. Verwendung der Verbindungen der Formel I gemäß den Ansprüchen 1 bis 15 zur Herstellung eines Arzneimittels zur Behandlung und/oder Prävention von Zuständen, die mit erniedrigtem HDL-Spiegel verbunden sind.

23. Verwendung der Verbindungen der Formel I gemäß den Ansprüchen 1 bis 15 zur Herstellung eines Arzneimittels zur Herstellung eines Arzneimittels zur Behandlung und/oder Prävention von atherosklerotischen Erkrankungen.

24. Verwendung der Verbindungen der Formel I gemäß den Ansprüchen 1 bis 15 in Kombination mit mindestens einem weiteren Wirkstoff zur Herstellung eines Arzneimittels zur Behandlung und/oder Prävention von Störungen, bei denen Insulin Resistenz eine Rolle spielt.

25. Verfahren zur Herstellung eines Arzneimittels enthaltend eine oder mehrere der Verbindungen der Formel I gemäß den Ansprüchen 1 bis 15, **dadurch gekennzeichnet, dass** diese mit einem pharmazeutisch geeigneten Träger vermischt wird und diese Mischung in eine für die Verabreichung geeignete Form gebracht wird.

26. Verfahren zur Herstellung von Verbindungen der allgemeinen Formel I gemäß den Ansprüchen 1 bis 15, **dadurch gekennzeichnet, dass** Triazolopyridin-derivate der Formel II
a) mit Carbamoylchloriden der Formel III acyliert werden;
oder
b) in zwei Stufen zunächst mit Phosgen oder Äquivalenten wie Chlörcarbonsäuretrichlormethylester, Carbonsäureditrichlormethylester oder Chlorameisensäure-4-nitrophenylester und einem zweiten Schritt mit Aminen der Formel IV umgesetzt werden,
worin die Substituenten die oben genannten Bedeutungen haben, und gegebenenfalls die Regioisomere durch bekannte Methoden getrennt werden.

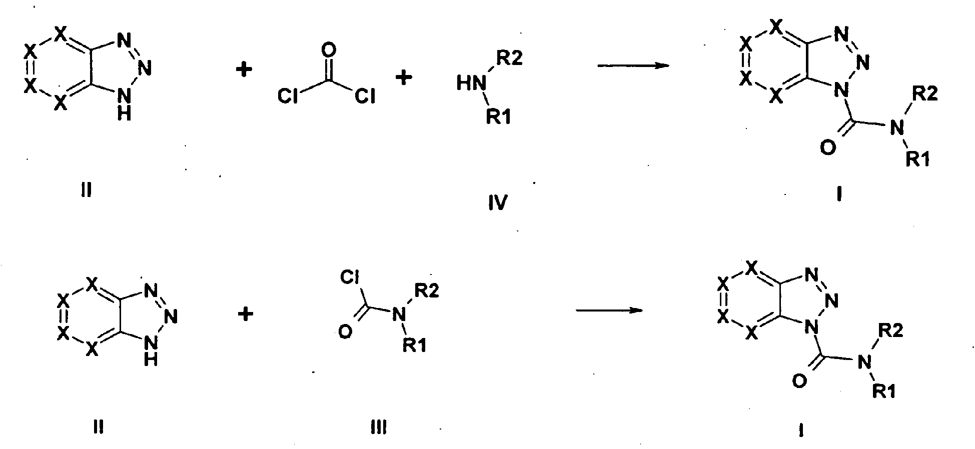

27. Verfahren zur Herstellung von Verbindungen der allgemeinen Formel I mit R2 Wasserstoff gemäß den Ansprüchen 1 bis 5 und 11 bis 13, **dadurch gekennzeichnet, dass** Triazolopyridin-derivate der Formel II mit Isocyanaten der Formel V: O=C=N-R1 umgesetzt werden, und gegebenenfalls die Regioisomere durch bekannte Methoden getrennt werden.

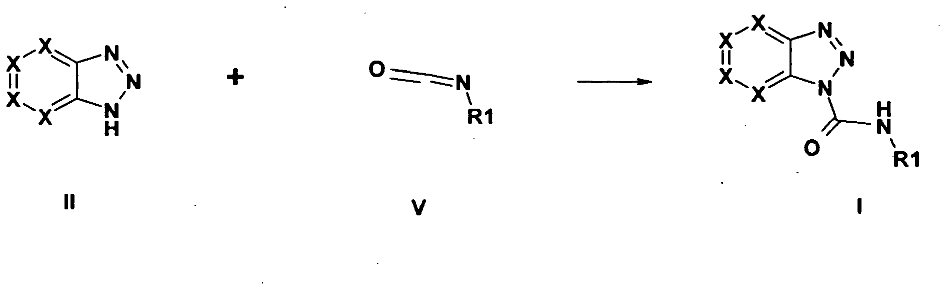

## Claims

1. A compound of the formula I 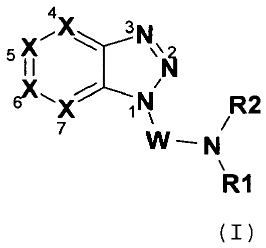 in which the meanings are:
X identically or differently =C(-R)- or =N-, where one X is =N-;
W -(C=O)-, -(S=O)-, -(SO₂)-;
R identically or differently hydrogen, halogen, (C₁-C₆) -alkyl, (C₁-C₃) -haloalkyl, (C₁-C₃) - alkyloxy- (C₁-C₃) -alkylene, hydroxy,_{'} phenoxy, amino, (C₁-C₆) -alkylamino, di- (C₂-C₁₂) - alkylamino, cyano, nitro, COOR3, mono-(C₁-C₆)-alkylaminocarbonyl, di(C₂-C₈)-alkylaminocarbonyl, (C₁-C₆)-alkylmercapto, (C₁-C₆) -alkylsulfinyl, (C₁-C₆) -alkylsulfonyl, aminosulfonyl, pentafluorosulfanyl, aryl, heteroaryl, (C₃-C₁₂) -cycloalkyl, (C₁-C₆) - alkylcarbonyl, O-CO-NR3R3a, O-CO-(C₁-C₆)-alkylene-CO-O- (C₁-C₆) -alkyl, O-CO- (C₁-C₆) - alkylene-CO-OH, O-CO-(C₁-C₆)-alkylene-CO-NR3R3a or unsubstituted or mono- or poly-F-substituted (C₁-C₆) -alkyloxy;
R1 (C₅-C₁₆) -alkyl, (C₅-C₁₂) -cycloalkyl, Y-aryl, Y-heteroaryl, Y-(C₅-C₁₂)-cycloalkyl, bicycle, where aryl, heteroaryl, cycloalkyl or bicycle may be substituted one or more times by halogen, (C₁-C₆) -alkyl, (C₁-C₆) -alkyloxy, hydroxy, (C₁-C₆)-alkylmercapto, amino, (C₁-C₆)-alkylamino, di-(C₂-C₁₂)-alkylamino, mono-(C₁-C₆)-alkylaminocarbonyl, di-(C₂-C₈)-alkylaminocarbonyl, (C₁-C₆)-alkyloxycarbonyl, (C₁-C₆)-alkylcarbonyl, cyano, nitro, trifluoromethyl, trifluoromethyloxy, (C₁-C₆) - alkylsulfonyl, aminosulfonyl;
Y (C₁-C₃)-alkylene which may be substituted one or more times by halogen, (C₁-C₂) -alkyl or hydroxy;
R2 hydrogen, (C₄-C₁₂) -alkyl; or
R1 and R2 together with the nitrogen atom carrying them may form a monocyclic, saturated or partly unsaturated 4- to 7-membered ring system or a bicyclic saturated or partly unsaturated 8-to 14 membered ring system whose individual members of the ring systems may be replaced by one to three atoms or atomic groups from the series -CHR4-, -CR4R5-, =(C-R4)-, -NR5-, -C(=O)-, -O-, -S-, -SO-, -SO₂-, with the proviso that two units from the series -0-, - S-, -SO-, -SO₂- may not be adjacent;
R3, R3a identically or differently hydrogen, (C₁-C₆)-alkyl, benzyl;
R4, R5 identically or differently (C₁-C₁₂)-alkyl, (C₁-C₆) -alkyloxy, halogen, (C₁-C₃) -haloalkyl, (C₁-C₃)-haloalkyloxy, COOR3, nitro, cyano, aryl, heterocycle, (C₃-C₁₂) -cycloalkyl, (C₁-C₃)-alkylene-aryl, (C₁-C₃)-alkylene-heterocycle, (C₁-C₃) -alkylene-cycloalkyl, where aryl, heterocycle or cycloalkyl may be substituted one or more times by halogen, (C₁-C₆)-alkyl, (C₁-C₆) -alkyloxy, O- (C₁-C₃) -alkylene-aryl, trifluoromethyl, trifluoromethoxy, hydroxy, mono-(C₁-C₆)-alkylaminocarbonyl, di-(C₂-C₈)-alkylaminocarbonyl, CO-(C₁-C₆)-alkyl, CO-O-(C₁-C₆)-alkyl, NR3R3a;
the tautomeric forms of the compounds and their physiologically tolerated salts,
with the proviso that the compound with the meanings of X in position 4, 5 and 6 = C(-R)- and in position 7 =N-, W is -(C=O)-, R is H and R1 and R2 form a morpholine, is excluded.

2. A compound of the formula I as claimed in claim 1, wherein
X is identically or differently =C(-R)- or =N-, where one X is =N-;
W is -(C=O)-.

3. A compound of the formula I as claimed in claim 1, wherein
X is identically or differently =C(-R)- or =N-, where one X is =N-;
W is - (C=O) -;
R is identically or differently hydrogen, halogen, (C₁-C₆)-alkyl, trifluoromethyl, (C₁-C₃) -alkyloxy- (C₁-C₃) -alkylene, hydroxy, phenoxy, amino, (C₁-C₆)-alkylamino, di-(C₂-C₁₂)-alkylamino, cyano, nitro, COOR3, mono-(C₁-C₆) -alkylaminocarbonyl, di- (C₂-C₈) -alkylaminocarbonyl, (C₁-C₆)-alkylsulfonyl, aminosulfonyl, pentafluorosulfanyl, phenyl, (C₁-C₆) -alkylcarbonyl, O-CO-NR3R3a, O-CO-(C₁-C₆) -alkylene-CO-O- (C₁-C₆) -alkyl, O-CO- (C₁-C₆)-alkylene-CO-OH, O-CO-(C₁-C₆)-alkylene-CO-NR3R3a or unsubstituted or mono- or poly-F-substituted (C₁-C₆) -alkyloxy;
R1 is (C₆-C₁₂) -alkyl, (C₅-C₁₂) -cycloalkyl, Y-phenyl, Y-heteroaryl, bicycle, where phenyl, heteroaryl, cycloalkyl or bicycle may be substituted one or more times by halogen, (C₁-C₆) -alkyl, (C₁-C₆) -alkyloxy, hydroxy, (C₁-C₆)-alkylmercapto, amino, (C₁-C₆)-alkylamino, di-(C₂-C₁₂)-alkylamino, mono-(C₁-C₆)-alkylaminocarbonyl, di-(C₂-C₈)-alkylaminocarbonyl, (C₁-C₆)-alkyloxycarbonyl, (C₁-C₆)-alkylcarbonyl, cyano, nitro, trifluoromethyl, trifluoromethyloxy, (C₁-C₆)-alkylsulfonyl, aminosulfonyl;
Y is -CH₂-, which may be substituted once by fluorine, methyl or hydroxy;
R2 is hydrogen, (C₅-C₁₀) -alkyl; or
R1 and R2 together with the nitrogen atom carrying them may form a monocyclic, saturated or partly unsaturated 5- to 6-membered ring system or a bicyclic saturated or partly unsaturated 9-to 10-membered ring system whose individual members of the ring systems may be replaced by one to three atoms or atomic groups from the series -CHR4-, -CR4R5-, =(C-R4)-, -NR5-, -C(=O)-, -0-, -S-, -SO₂-, with the proviso that two units from the series -O-, -S-, -SO₂-may not be adjacent;
R3, R3a are identically or differently hydrogen, (C₁-C₆)-alkyl, benzyl;
R4, R5 are identically or differently (C₁-C₁₀)-alkyl, (C₁-C₆) -alkyloxy, halogen, (C₁-C₃) -haloalkyl, (C₁-C₃)-haloalkyloxy, COOR3, nitro, cyano, aryl, heterocycle, (C₃-C₇)-cycloalkyl, (C₁-C₃) - alkylene-aryl, (C₁-C₃)-alkylene-heteroaryl, where aryl, heterocycle or cycloalkyl may be substituted one or more times by halogen, (C₁-C₆₎ -alkyl, (C₁-C₆) -alkyloxy, -O- (C₁-C₃) - alkylene-aryl, trifluoromethyl, trifluoromethoxy, hydroxy, mono-(C₁-C₆)-alkylaminocarbonyl, di- (C₂-C₈) - alkylaminocarbonyl, CO-(C₁-C₆)-alkyl, CO-O-(C₁-C₆)-alkyl, NR3R3a;
the tautomeric forms of the compounds and their physiologically tolerated salts.

4. A compound of the formula I as claimed in claims 1 or 3, wherein
X is identically or differently =C(-R)- or =N-, where one X is =N-;
W is -(C=O)-;
R is identically or differently hydrogen, halogen, (C₁-C₆) -alkyl, trifluoromethyl, hydroxy, amino, cyano, COOR3, mono-(C₁-C₆)-alkylaminocarbonyl, di- (C₂-C₈) - alkylaminocarbonyl, (C₁-C₆)-alkylsulfonyl, aminosulfonyl, pentafluorosulfanyl, (C₁-C₆)-alkylcarbonyl, O-CO-NR3R3a, O-(O-(C₁-C₆)-alkylene-CO-O-(C₁-C₆)-alkyl or unsubstituted or mono- or poly-F-substituted (C₁-C₆)-alkyloxy;
R1 is (C₆-C₁₂)-alkyl, Y-phenyl, Y-heteroaryl, bicycle, where aryl, heteroaryl or bicycle may be substituted one or more times by halogen, (C₁-C₆)-alkyl, (C₁-C₆)-alkyloxy, hydroxy, amino, (C₁-C₆)-alkylamino, di-(C₂-C₁₂)-alkylamino, mono- (C₁-C₆) - alkylaminocarbonyl, di- (C₂-C₈) - alkylaminocarbonyl, (C₁-C₆)-alkyloxycarbonyl, (C₁-C₆)-alkylcarbonyl, cyano, trifluoromethyl, trifluoromethyloxy, (C₁-C₆) -alkylsulfonyl, aminosulfonyl;
Y is -CH₂-;
R2 is hydrogen, (C₅-C₁₀)-alkyl; or
R1 and R2 together with the nitrogen atom carrying them may form a monocyclic, saturated or partly unsaturated 5- to 6-membered ring system or a bicyclic saturated or partly unsaturated 9-to 10-membered ring system whose individual members of the ring systems may be replaced by one to three atoms or atomic groups from the series -CHR4-, -CR4R5-, =(C-R4)-, -NR5-, -C(=O)-, -0-, -S-, with the proviso that two units from the series -O-, -S-, may not be adjacent;
R3, R3a are identically or differently hydrogen, (C₁-C₆)-alkyl, benzyl;
R4, R5 are identically or differently (C₁-C₁₀)-alkyl, (C₁-C₆) -alkyloxy, halogen, (C₁-C₃)-haloalkyl, (C₁-C₃)-haloalkyloxy, COOR3, nitro, cyano, aryl, heteroaryl, (C₃-C₇) -cycloalkyl, (C₁-C₃) - alkylene-aryl, (C₁-C₃)-alkylene-heteroaryl, where aryl, heteroaryl or cycloalkyl may be substituted one or more times by halogen, (C₁-C₆) -alkyl, (C₁-C₆) -alkyloxy, O- (C₁-C₃) - alkylene-aryl, trifluoromethyl, trifluoromethoxy, hydroxy, mono-(C₁-C₆)-alkylaminocarbonyl, di- (C₂-C₈) - alkylaminocarbonyl, CO-(C₁-C₆)-alkyl, CO-O-(C₁-C₆) -alkyl, amino;
the tautomeric forms of the compounds and their physiologically tolerated salts.

5. A compound of the formula I as claimed in claims 1 to 4, wherein
X is identically or differently =C(-R)- or =N-, where one X is =N-;
W is - (C=O) -;
R is identically or differently hydrogen, halogen, (C₁-C₆)-alkyl, trifluoromethyl, hydroxy, amino, cyano, COOR3, (C₁-C₆)-alkylsulfonyl, aminosulfonyl, (C₁-C₆)-alkylcarbonyl or unsubstituted or mono- or poly-F-substituted (C₁-C₆) -alkyloxy;
R1 is (C₆-C₁₂)-alkyl, Y-phenyl, where phenyl may be substituted one to three times by halogen, (C₁-C₆) -alkyl, (C₁-C₆) -alkyloxy, hydroxy, amino, (C₁-C₆)-alkyloxycarbonyl, (C₁-C₆)-alkylcarbonyl, cyano, trifluoromethyl, trifluoromethyloxy, (C₁-C₆)-alkylsulfonyl, aminosulfonyl;
Y is -CH₂-;
R2 is hydrogen, (C₅-C₁₀)-alkyl; or
R1 and R2 together with the nitrogen atom carrying them may form a monocyclic, saturated 5- to 6-membered ring system or a bicyclic saturated or partly unsaturated 9- to 10-membered ring system whose individual members of the ring systems may be replaced by one to three atoms or atomic groups from the series -CHR4-, - CR4R5-, =(C-R4)-, -NR5-, -C(=O)-, -O-, -S-, with the proviso that two units from the series -0-, -S- may not be adjacent;
R3, R3a are identically or differently hydrogen, (C₁-C₆)-alkyl;
R4, R5 are identically or differently (C₁-C₁₀)-alkyl, (C₁-C₆)-alkyloxy, halogen, trifluoromethyl, trifluoromethoxy, COOR3, cyano, aryl, cyclopropyl, (C₁-C₃)-alkylene-phenyl, where aryl may be substituted one or more times by halogen, (C₁-C₆) -alkyl, (C₁-C₆) -alkyloxy, 0-(C₁-C₃)-alkylene-phenyl, trifluoromethyl, trifluoromethoxy, hydroxy, mono-(C₁-C₆)-alkylaminocarbonyl, di- (C₂-C₈) - alkylaminocarbonyl, CO-(C₁-C₆)-alkyl, CO-O-(C₁-C₆) -alkyl, amino;
the tautomeric forms of the compounds and their physiologically tolerated salts.

6. A compound of the formula I as claimed in claims 1 to 5, wherein
NR1R2 is a monocyclic saturated 5- to 6-membered ring system which comprises in position 4 an atom or atomic member from the series -CHR4-, -CR4R5-, -NR5-, -(C=O) or is a bicyclic saturated or partly unsaturated 9- to 10-membered ring system whose individual members of the ring system may be replaced by one or two atomic groups from the series -CHR4-, -(C=R4)-, =(C-R4)-, -NR5-, -(C=O)-.

7. A compound of the formula I as claimed in claims 1 to 6, wherein
R1 and R2 together with the nitrogen atom carrying them form a monocyclic, saturated 5- to 6-membered ring system or a bicyclic saturated or partly unsaturated 9-to 10-membered ring system, whose individual members of the ring systems may be replaced by an atomic group from the series -CHR4-, -NR5.

8. A compound of the formula I as claimed in claims 1 to 7, wherein
X is identically or differently =C(-R)- or =N-, where one X is =N-;
R is identically or differently hydrogen, Cl;
NR1R2 is piperidine which comprises in position 4 the atomic member -CHR4-; or is piperazine which comprises in position 4 the atomic member -NR5;
R4, R5 are (C₁-C₈)-alkyl, phenyl, -(C₁-C₂)-alkylenephenyl, naphthyl, pyrrolidin-2-one, benzofuranyl, where phenyl may be substituted once, twice or three times by methyl, ethyl, t-butyl, methoxy, ethoxy, F, Cl, Br, OH, CF₃, NH₂, CO-OCH₃, CO-CH₃, phenyl, -O-benzyl.

9. A compound of the formula I as claimed in claims 1 to 8, wherein
NR1R2 is piperidine which comprises in position 4 the atomic member -CHR4-, or is piperazine which comprises in position 4 the atomic member -NR5;
R4, R5 are methyl or phenyl which may be substituted once or twice by methyl, methoxy, ethoxy, F, Cl, Br, OH, CF₃, NH₂, CO-OCH₃, CO-CH₃, phenyl, -O-benzyl.

10. A compound of the formula I as claimed in claims 1 to 5, wherein
X is identically or differently = C(-R-)- or =N-, where one X is =N-;
R is identically or differently hydrogen, Cl;
NR1R2 is tetrahydroquinoline or tetrahydroisoquinoline which may be substituted once or twice by methoxy.

11. A compound of the formula I as claimed in claims 1 to 5, wherein
R2 is hydrogen.

12. A compound of the formula I as claimed in claims 1 to 5, wherein
R1 is (C₆-C₁₂) -alkyl, Y-phenyl, where phenyl may be substituted one to three times by halogen, (C₁-C₆)-alkyl, (C₁-C₆)-alkyloxy, hydroxy, amino, (C₁-C₆) -alkyloxycarbonyl, (C₁-C₆) - alkylcarbonyl, cyano, trifluoromethyl, trifluoromethyloxy, (C₁-C₆)-alkylsulfonyl, aminosulfonyl;
Y is -CH₂-;
R2 is hydrogen.

13. A compound of the formula I as claimed in claims 1 to 5, wherein
X is identically or differently =C(-R)- or =N-, where one X is =N-;
R is identically or differently hydrogen, Cl;
R1 is hexyl, Y-phenyl, where phenyl may be substituted by methyl, preferably in position 2;
Y is -CH₂-;
R2 is hydrogen.

14. A compound of the formula I as claimed in claims 1 to 13, wherein
X in position 4, 5 and 6 is identically or differently =C(-R)-, and in position 7 is =N-

15. A compound of the formula I, as claimed in claims 1 to 13, wherein
X optionally in position 4 or 7 is =C(-R)- with R = hydrogen or =N-, and in position 5 or 6 is =C(-R)- with R not hydrogen.

16. A medicament comprising one or more compounds of the formula I as claimed in claims 1 to 15.

17. The use of the compounds of the formula I as claimed in claims 1 to 15 for the production of a medicament for the treatment and/or prevention of disorders of fatty acid metabolism and glucose utilization disorders.

18. The use of the compounds of the formula I as claimed in claims 1 to 15 for the production of a medicament for the treatment and/or prevention of disorders in which insulin resistance is involved.

19. The use of the compounds of the formula I as claimed in claims 1 to 15 for the production of a medicament for the treatment and/or prevention of diabetes mellitus and the sequelae associated therewith.

20. The use of the compounds of the formula I as claimed in claims 1 to 15 for the production of a medicament for the treatment and/or prevention of dyslipidemias and their sequelae.

21. The use of the compounds of the formula I as claimed in claims 1 to 15 for the production of a medicament for the treatment and/or prevention of conditions associated with the metabolic syndrome.

22. The use of the compounds of the formula I as claimed in claims 1 to 15 for the production of a medicament for the treatment and/or prevention of conditions associated with a reduced HDL level.

23. The use of the compounds of the formula I as claimed in claims 1 to 15 for the production of a medicament for the treatment and/or prevention of atherosclerotic disorders.

24. The use of the compounds of the formula I as claimed in claims 1 to 15 in combination with at least one further active ingredient for the production of a medicament for the treatment and/or prevention of disorders in which insulin resistance is involved.

25. A process for producing a medicament comprising one or more of the compounds of the formula I as claimed in claims 1 to 15, which comprises mixing the latter with a pharmaceutically suitable carrier and converting this mixture into a form suitable for administration.

26. A process for preparing compounds of the formula I as claimed in claims 1 to 15, which comprises triazolopyridine derivatives of the formula II
a) being acylated with carbamoyl chlorides of the formula III;
or
b) in two stages being reacted firstly with phosgene or equivalents such as trichloromethyl chlorocarbonate, ditrichloromethyl carbonate or 4-nitrophenyl chloroformate and in a second step with amines of the formula IV,
in which the substituents have the abovementioned meanings, and, where appropriate, separating the regioisomers by known methods.

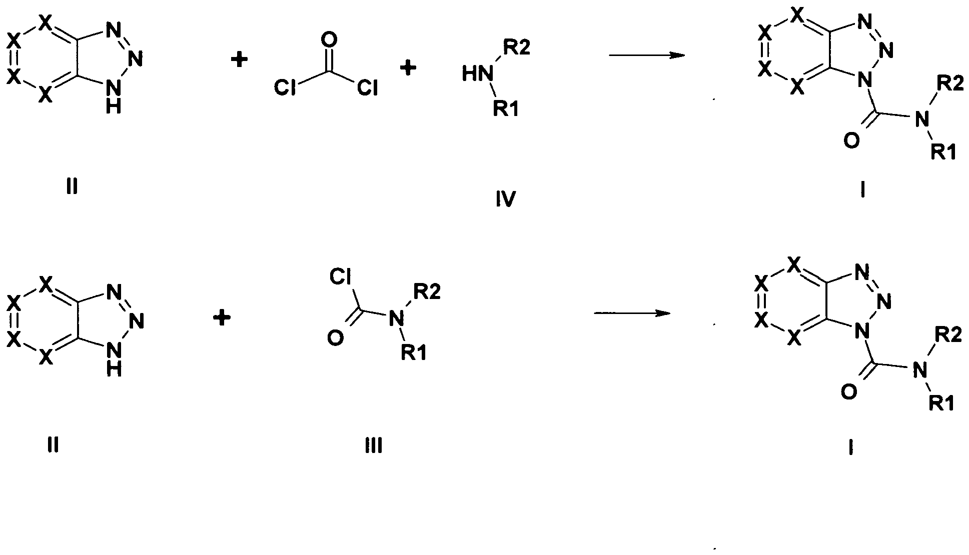

27. A process for preparing compounds of the formula I with R2 hydrogen as claimed in claims 1 to 5 and 11 to 13, which comprises triazolopyridine derivatives of the formula II being reacted with isocyanates of the formula V: O=C=N-R1, and, where appropriate, the regioisomers being separated by known methods.

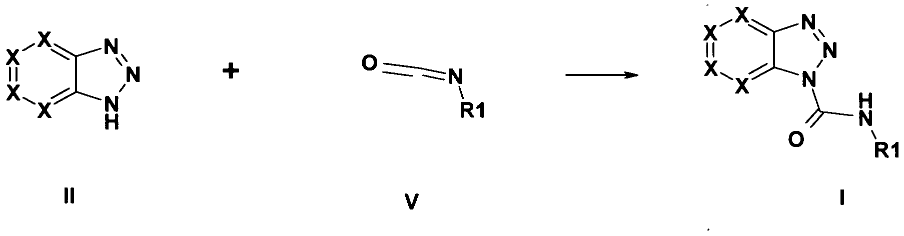

## Revendications

1. Composé de formule générale I 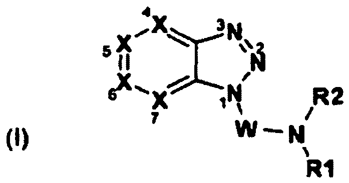 dans laquelle :
X sont identiques ou différents et signifient =C(-R)- ou =N-, un X représentant =N- ;
W signifie -(C=O)-, -(S=O)-, -(SO₂)- ;
R sont identiques ou différents et signifient hydrogène, halogène, alkyle en (C₁-C₆), haloalkyle en (C₁-C₃), alkyloxy en (C₁-C₃)-alkylène en (C₁-C₃), hydroxy, phénoxy, amino, alkylamino en (C₁-C₆), di-alkylamino en (C₂-C₁₂), cyano, nitro, COOR3, mono-alkylaminocarbonyle en (C₁-C₆), di-alkylaminocarbonyle en (C₂-C₈), alkylmercapto en (C₁-C₆), alkylsulfinyle en (C₁-C₆), alkylsulfonyle en (C₁-C₆), aminosulfonyle, pentafluorosulfanyle, aryle, hétéroaryle, cycloalkyle en (C₃-C₁₂), alkylcarbonyle en (C₁-C₆), O-CO-NR3R3a, 0-CO-alkylène en (C₁-C₆)-CO-O-alkyle en (C₁-C₆), O-CO-alkylène en (C₁-C₆) -CO-OH, O-CO-alkylène en (C₁-C₆) -CO-NR3R3a ; ou alkyloxy en (C₁-C₆) non substitué ou substitué une ou plusieurs fois par F ;
R1 signifie alkyle en (C₅-C₁₆), cycloalkyle en (C₅-C₁₂), Y-aryle, Y-hétéroaryle, Y-cycloalkyle en (C₅-C₁₂), bicycle ; aryle, hétéroaryle, cycloalkyle ou bicycle pouvant être substitué une ou plusieurs fois par halogène, alkyle en (C₁-C₆), alkyloxy en (C₁-C₆), hydroxy, alkylmercapto en (C₁-C₆), amino, alkylamino en (C₁-C₆), di-alkylamino en (C₂-C₁₂), mono-alkylaminocarbonyle en (C₁-C₆), di-alkylaminocarbonyle en (C₂-C₈), alkyloxycarbonyle en (C₁-C₆), alkylcarbonyle en (C₁-C₆), cyano, nitro, trifluorométhyle, trifluorométhyloxy, alkylsulfonyle en (C₁-C₆), aminosulfonyle ;
Y signifie alkylène en (C₁-C₃), qui peut être substitué une ou plusieurs fois par halogène, alkyle en (C₁-C₂) ou hydroxy ;
R2 signifie hydrogène, alkyle en (C₄-C₁₂) ; ou
R1 et R2 peuvent former ensemble avec l'atome d'azote qui les porte un système cyclique monocyclique saturé ou partiellement insaturé de 4 à 7 éléments ou un système cyclique bicyclique saturé ou partiellement insaturé de 8 à 14 éléments, dont les éléments individuels des systèmes cycliques peuvent être remplacés par un à trois atomes ou groupes d'atomes de la série -CHR4-, -CR4R5-, =(C-R4)-, -NR5-, -C(=O)-, -0-, -S-, -SO-, -SO₂-, à condition que deux unités de la série -O-, -S-, -SO-, -SO₂- ne puissent pas être voisines ;
R3, R3a sont identiques ou différents et signifient hydrogène, alkyle en (C₁-C₆), benzyle ;
R4, R5 sont identiques ou différents et signifient alkyle en (C₁-C₁₂), alkyloxy en (C₁-C₆), halogène, haloalkyle en (C₁-C₃), haloalkyloxy en (C₁-C₃), COOR3, nitro, cyano, aryle, hétérocycle, cycloalkyle en (C₃-C₁₂), alkylène en (C₁-C₃) -aryle, alkylène en (C₁-C₃)-hétérocycle, alkylène en (C₁-C₃)-cycloalkyle ; aryle, hétérocycle ou cycloalkyle pouvant être substitué une ou plusieurs fois par halogène, alkyle en (C₁-C₆), alkyloxy en (C₁-C₆), O-alkylène en (C₁-C₃)-aryle, trifluorométhyle, trifluorométhoxy, hydroxy, mono-alkylaminocarbonyle en (C₁-C₆), di-alkylaminocarbonyle en (C₂-C₈), CO-alkyle en (C₁-C₆), CO-O-alkyle en (C₁-C₆), NR3R3a ;
les formes tautomères des composés, ainsi que leurs sels physiologiquement compatibles,
à condition que le composé dans lequel X signifie à la position 4, 5 et 6 =C(-R)-, à la position 7 =N-, W est -(C=O)-, R est H et R1 et R2 forment une morpholine soit exclu.

2. Composé de formule I selon la revendication 1, **caractérisé en ce que**
X sont identiques ou différents et signifient =C(-R)- ou =N-, un X représentant =N- ;
W signifie -(C=O)-.

3. Composé de formule I selon la revendication 1, **caractérisé en ce que**
X sont identiques ou différents et signifient =C(-R)- ou =N-, un X représentant =N- ;
W signifie -(C=O)- ;
R sont identiques ou différents et signifient hydrogène, halogène, alkyle en (C₁-C₆), trifluorométhyle, alkyloxy en (C₁-C₃)-alkylène en (C₁-C₃), hydroxy, phénoxy, amino, alkylamino en (C₁-C₆), di-alkylamino en (C₂-C₁₂), cyano, nitro, COOR3, mono-alkylaminocarbonyle en (C₁-C₆), di-alkylaminocarbonyle en (C₂-C₈), alkylsulfonyle en (C₁-C₆), aminosulfonyle, pentafluorosulfanyle, phényle, alkylcarbonyle en (C₁-C₆), O-CO-NR3R3a, O-CO-alkylène en (C₁-C₆)-CO-O-alkyle en (C₁-C₆), O-CO-alkylène en (C₁-C₆)-CO-OH, O-CO-alkylène en (C₁-C₆) -CO-NR3R3a ; ou alkyloxy en (C₁-C₆) non substitué ou substitué une ou plusieurs fois par F ;
R1 signifie alkyle en (C₆-C₁₂), cycloalkyle en (C₅-C₁₂), Y-phényle, Y-hétéroaryle, bicycle ; phényle, hétéroaryle, cycloalkyle ou bicycle pouvant être substitué une ou plusieurs fois par halogène, alkyle en (C₁-C₆), alkyloxy en (C₁-C₆), hydroxy, alkylmercapto en (C₁-C₆), amino, alkylamino en (C₁-C₆), di-alkylamino en (C₂-C₁₂), mono-alkylaminocarbonyle en (C₁-C₆), di-alkylaminocarbonyle en (C₂-C₈), alkyloxycarbonyle en (C₁-C₆), alkylcarbonyle en (C₁-C₆), cyano, nitro, trifluorométhyle, trifluorométhyloxy, alkylsulfonyle en (C₁-C₆), aminosulfonyle ;
Y signifie -CH₂-, qui peut être substitué une fois par fluor, méthyle ou hydroxy ;
R2 signifie hydrogène, alkyle en (C₅-C₁₀) ; ou
R1 et R2 peuvent former ensemble avec l'atome d'azote qui les porte un système cyclique monocyclique saturé ou partiellement insaturé de 5 à 6 éléments ou un système cyclique bicyclique saturé ou partiellement insaturé de 9 à 10 éléments, dont les éléments individuels des systèmes cycliques peuvent être remplacés par un à trois atomes ou groupes d'atomes de la série -CHR4-, -CR4R5-, =(C-R4)-, -NR5-, -C(=O)-, -0-, -S-, -SO₂-, à condition que deux unités de la série - 0-, -S-, -SO₂- ne puissent pas être voisines ;
R3, R3a sont identiques ou différents et signifient hydrogène, alkyle en (C₁-C₆), benzyle ;
R4, R5 sont identiques ou différents et signifient alkyle en (C₁-C₁₀), alkyloxy en (C₁-C₆), halogène, haloalkyle en (C₁-C₃), haloalkyloxy en (C₁-C₃), COOR3, nitro, cyano, aryle, hétérocycle, cycloalkyle en (C₃-C₇), alkylène en (C₁-C₃)-aryle, alkylène en (C₁-C₃)-hétéroaryle ; aryle, hétérocycle ou cycloalkyle pouvant être substitué une ou plusieurs fois par halogène, alkyle en (C₁-C₆), alkyloxy en (C₁-C₆), O-alkylène en (C₁-C₃)-aryle, trifluorométhyle, trifluorométhoxy, hydroxy, mono-alkylaminocarbonyle en (C₁-C₆), di-alkylaminocarbonyle en (C₂-C₈), CO-alkyle en (C₁-C₆), CO-O-alkyle en (C₁-C₆), NR3R3a ;
les formes tautomères des composés, ainsi que leurs sels physiologiquement compatibles.

4. Composé de formule I selon les revendications 1 ou 3, **caractérisé en ce que**
X sont identiques ou différents et signifient =C(-R)- ou =N-, un X représentant =N- ;
W signifie -(C=O)- ;
R sont identiques ou différents et signifient hydrogène, halogène, alkyle en (C₁-C₆), trifluorométhyle, hydroxy, amino, cyano, COOR3, mono-alkylaminocarbonyle en (C₁-C₆), di-alkylaminocarbonyle en (C₂-C₈), alkylsulfonyle en (C₁-C₆), aminosulfonyle, pentafluorosulfanyle, alkylcarbonyle en (C₁-C₆), O-CO-NR3R3a, O-CO-alkylène en (C₁-C₆) -CO-O-alkyle en (C₁-C₆) ;
ou alkyloxy en (C₁-C₆) non substitué ou substitué une ou plusieurs fois par F ;
R1 signifie alkyle en (C₆-C₁₂), Y-phényle, Y-hétéroaryle, bicycle ; aryle, hétéroaryle ou bicycle pouvant être substitué une ou plusieurs fois par halogène, alkyle en (C₁-C₆), alkyloxy en (C₁-C₆), hydroxy, amino, alkylamino en (C₁-C₆), di-alkylamino en (C₂-C₁₂), mono-alkylaminocarbonyle en (C₁-C₆), di-alkylaminocarbonyle en (C₂-C₈), alkyloxycarbonyle en (C₁-C₆) , alkylcarbonyle en (C₁-C₆) , cyano, trifluorométhyle, trifluorométhyloxy, alkylsulfonyle en (C₁-C₆), aminosulfonyle ;
Y signifie -CH₂- ;
R2 signifie hydrogène, alkyle en (C₅-C₁₀) ; ou
R1 et R2 peuvent former ensemble avec l'atome d'azote qui les porte un système cyclique monocyclique saturé ou partiellement insaturé de 5 à 6 éléments ou un système cyclique bicyclique saturé ou partiellement insaturé de 9 à 10 éléments, dont les éléments individuels des systèmes cycliques peuvent être remplacés par un à trois atomes ou groupes d'atomes de la série -CHR4-, -CR4R5-, =(C-R4)-, -NR5-, -C (=O) -, -0-, -S-, à condition que deux unités de la série -O-, -S-ne puissent pas être voisines ;
R3, R3a sont identiques ou différents et signifient hydrogène, alkyle en (C₁-C₆), benzyle ;
R4, R5 sont identiques ou différents et signifient alkyle en (C₁-C₁₀), alkyloxy en (C₁-C₆), halogène, haloalkyle en (C₁-C₃), haloalkyloxy en (C₁-C₃), COOR3, nitro, cyano, aryle, hétéroaryle, cycloalkyle en (C₃-C₇), alkylène en (C₁-C₃) -aryle, alkylène en (C₁-C₃) - hétéroaryle ; aryle, hétéroaryle ou cycloalkyle pouvant être substitué une ou plusieurs fois par halogène, alkyle en (C₁-C₆) , alkyloxy en (C₁-C₆) , O-alkylène en (C₁-C₃)-aryle, trifluorométhyle, trifluorométhoxy, hydroxy, mono-alkylaminocarbonyle en (C₁-C₆), di-alkylaminocarbonyle en (C₂-C₈), CO-alkyle en (C₁-C₆), CO-O-alkyle en (C₁-C₆) , amino ;
les formes tautomères des composés, ainsi que leurs sels physiologiquement compatibles.

5. Composé de formule I selon les revendications 1 à 4, **caractérisé en ce que**
X sont identiques ou différents et signifient =C(-R)- ou =N-, un X représentant =N- ;
W signifie -(C=O)- ;
R sont identiques ou différents et signifient hydrogène, halogène, alkyle en (C₁-C₆), trifluorométhyle, hydroxy, amino, cyano, COOR3, alkylsulfonyle en (C₁-C₆), aminosulfonyle, alkylcarbonyle en (C₁-C₆) ; ou alkyloxy en (C₁-C₆) non substitué ou substitué une ou plusieurs fois par F ;
R1 signifie alkyle en (C₆-C₁₂), Y-phényle ; phényle pouvant être substitué une à trois fois par halogène, alkyle en (C₁-C₆), alkyloxy en (C₁-C₆), hydroxy, amino, alkyloxycarbonyle en (C₁-C₆), alkylcarbonyle en (C₁-C₆), cyano, trifluorométhyle, trifluorométhyloxy, alkylsulfonyle en (C₁-C₆), aminosulfonyle ;
Y signifie -CH₂- ;
R2 signifie hydrogène, alkyle en (C₅-C₁₀) ; ou
R1 et R2 peuvent former ensemble avec l'atome d'azote qui les porte un système cyclique monocyclique saturé de 5 à 6 éléments ou un système cyclique bicyclique saturé ou partiellement insaturé de 9 à 10 éléments, dont les éléments individuels des systèmes cycliques peuvent être remplacés par un à trois atomes ou groupes d'atomes de la série -CHR4-, -CR4R5-, =(C-R4)-, -NR5-, -C(=O)-, -O-, -S-, à condition que deux unités de la série -O-, -S- ne puissent pas être voisines ;
R3, R3a sont identiques ou différents et signifient hydrogène, alkyle en (C₁-C₆) ;
R4, R5 sont identiques ou différents et signifient alkyle en (C₁-C₁₀), alkyloxy en (C₁-C₆), halogène, trifluorométhyle, trifluorométhoxy, COOR3, cyano, aryle, cyclopropyle, alkylène en (C₁-C₃) -phényle ; aryle pouvant être substitué une ou plusieurs fois par halogène, alkyle en (C₁-C₆), alkyloxy en (C₁-C₆), 0-alkylène en (C₁-C₃)-phényle, trifluorométhyle, trifluorométhoxy, hydroxy, mono-alkylaminocarbonyle en (C₁-C₆), di-alkylaminocarbonyle en (C₂-C₈), CO-alkyle en (C₁-C₆), CO-O-alkyle en (C₁-C₆), amino ;
les formes tautomères des composés, ainsi que leurs sels physiologiquement compatibles.

6. Composé de formule I selon les revendications 1 à 5, **caractérisé en ce que**
NR1R2 représente un système cyclique monocyclique saturé de 5 à 6 éléments, qui contient en position 4 un atome ou groupe d'atomes de la série -CHR4-, -CR4R5-, - NR5-, -C(=O)-, ou un système cyclique bicyclique saturé ou partiellement insaturé de 9 à 10 éléments, dont les éléments individuels du système cyclique peuvent être remplacés par un ou deux groupes d'atomes de la série - CHR4-, -(C=R4)-, =(C-R4)-, -NR5-, -C (=O) -.

7. Composé de formule I selon les revendications 1 à 6, **caractérisé en ce que**
R1 et R2 peuvent former ensemble avec l'atome d'azote qui les porte un système cyclique monocyclique saturé de 5 à 6 éléments ou un système cyclique bicyclique saturé ou partiellement insaturé de 9 à 10 éléments, dont les éléments individuels des systèmes cycliques peuvent être remplacés par un groupe d'atomes de la série -CHR4-, -NR5-.

8. Composé de formule I selon les revendications 1 à 7, **caractérisé en ce que**
X sont identiques ou différents et signifient =C(-R)- ou =N-, un X représentant =N- ;
R sont identiques ou différents et signifient hydrogène, Cl ;
NR1R2 représente pipéridine qui contient en position 4 le groupe d'atomes -CHR4- ; ou
représente pipérazine qui contient en position 4 le groupe d'atomes -NR5 ;
R4, R5 signifient alkyle en (C₁-C₈), phényle, alkylène en (C₁-C₂)-phényle, naphtyle, pyrrolidin-2-one, benzofuranyle ; phényle pouvant être substitué une, deux ou trois fois par méthyle, éthyle, t-butyle, méthoxy, éthoxy, F, Cl, Br, OH, CF₃, NH₂, CO-OCH₃, CO-CH₃, phényle, -O-benzyle.

9. Composé de formule I selon les revendications 1 à 8, **caractérisé en ce que**
NR1R2 représente pipéridine qui contient en position 4 le groupe d'atomes -CHR4- ; ou
représente pipérazine qui contient en position 4 le groupe d'atomes -NR5 ;
R4, R5 signifient méthyle ou phényle, qui peut être substitué une ou deux fois par méthyle, méthoxy, éthoxy, F, Cl, Br, OH, CF₃, NH₂, CO-OCH₃, CO-CH₃, phényle, -O-benzyle.

10. Composé de formule I selon les revendications 1 à 5, **caractérisé en ce que**
X sont identiques ou différents et signifient =C(-R)- ou =N-, un X représentant =N- ;
R sont identiques ou différents et signifient hydrogène, Cl ;
NR1R2 représente tétrahydroquinoline ou tétrahydroisoquinoline, qui peut être substitué une ou deux fois par méthoxy.

11. Composé de formule I selon les revendications 1 à 5, **caractérisé en ce que**
R2 signifie hydrogène.

12. Composé de formule I selon les revendications 1 à 5, **caractérisé en ce que**
R1 signifie alkyle en (C₆-C₁₂), Y-phényle ; phényle pouvant être substitué une à trois fois par halogène, alkyle en (C₁-C₆), alkyloxy en (C₁-C₆), hydroxy, amino, alkyloxycarbonyle en (C₁-C₆), alkylcarbonyle en (C₁-C₆), cyano, trifluorométhyle, trifluorométhyloxy, alkylsulfonyle en (C₁-C₆), aminosulfonyle ;
Y signifie -CH₂- ;
R2 signifie hydrogène.

13. Composé de formule I selon les revendications 1 à 5, **caractérisé en ce que**
X sont identiques ou différents et signifient =C(-R)- ou =N-, un X représentant =N- ;
R sont identiques ou différents et signifient hydrogène, Cl ;
R1 signifie hexyle, Y-phényle ; phényle pouvant être substitué par méthyle, de préférence en position 2 ;
Y signifie -CH₂- ;
R2 signifie hydrogène.

14. Composé de formule I selon les revendications 1 à 13, **caractérisé en ce que**
X représente en position 4, 5 et 6 =C(-R)-identiques ou différents, en position 7 =N-.

15. Composé de formule I selon les revendications 1 à 13, **caractérisé en ce que**
X représente éventuellement en position 4 ou 7 =C(-R)- avec R = hydrogène ou =N-, en position 5 ou 6 =C(-R)- avec R différent de l'hydrogène.

16. Médicament contenant un ou plusieurs composés de formule I selon les revendications 1 à 15.

17. Utilisation des composés de formule I selon les revendications 1 à 15 pour la fabrication d'un médicament pour le traitement et/ou la prévention des troubles du métabolisme des acides gras et des troubles de l'utilisation du glucose.

18. Utilisation des composés de formule I selon les revendications 1 à 15 pour la fabrication d'un médicament pour le traitement et/ou la prévention des troubles dans lesquels la résistance à l'insuline joue un rôle.

19. Utilisation des composés de formule I selon les revendications 1 à 15 pour la fabrication d'un médicament pour le traitement et/ou la prévention du diabète sucré et des complications associées.

20. Utilisation des composés de formule I selon les revendications 1 à 15 pour la fabrication d'un médicament pour le traitement et/ou la prévention des dyslipidémies et leurs conséquences.

21. Utilisation des composés de formule I selon les revendications 1 à 15 pour la fabrication d'un médicament pour le traitement et/ou la prévention des états associés avec le syndrome métabolique.

22. Utilisation des composés de formule I selon les revendications 1 à 15 pour la fabrication d'un médicament pour le traitement et/ou la prévention des états associés avec un niveau d'HDL abaissé.

23. Utilisation des composés de formule I selon les revendications 1 à 15 pour la fabrication d'un médicament pour le traitement et/ou la prévention des maladies athérosclérotiques.

24. Utilisation des composés de formule I selon les revendications 1 à 15 en combinaison avec au moins un agent actif supplémentaire pour la fabrication d'un médicament pour le traitement et/ou la prévention des troubles dans lesquels la résistance à l'insuline joue un rôle.

25. Procédé de fabrication d'un médicament contenant un ou plusieurs des composés de formule I selon les revendications 1 à 15, **caractérisé en ce que** ceux-ci sont mélangés avec un véhicule pharmaceutiquement approprié et ce mélange est mis sous une forme appropriée pour l'administration.

26. Procédé de fabrication de composés de formule générale I selon les revendications 1 à 15, **caractérisé en ce que** des dérivés de triazolopyridine de formule II
a) sont acylés avec des chlorures de carbamoyle de formule III ; ou
b) sont mis en réaction en deux étapes, tout d'abord avec du phosgène ou des équivalents tels que l'ester trichlorométhylique d'acide chlorocarboxyllique, l'ester ditrichlorométhylique d'acide carboxylique ou l'ester 4-nitrophénylique d'acide chloroformique et, lors d'une seconde étape, avec des amines de formule IV les substituants ayant les significations données précédemment, et les régioisomères sont éventuellement séparés par des procédés connus.

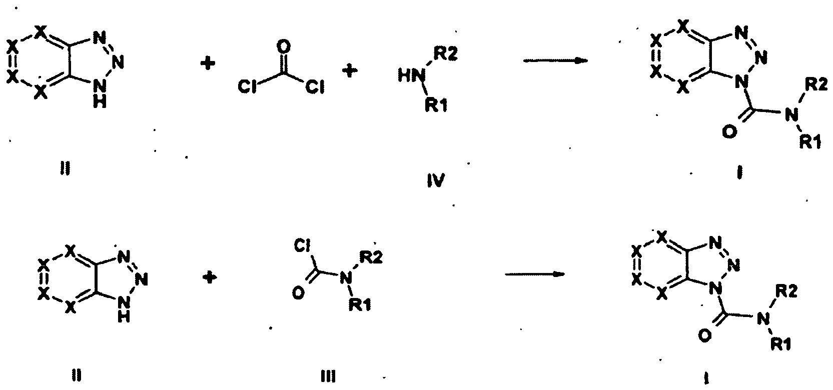

27. Procédé de fabrication de composés de formule générale I avec R2 hydrogène selon les revendications 1 à 5 et 11 à 13, **caractérisé en ce que** des dérivés de triazolopyridine de formule II sont mis en réaction avec des isocyanates de formule V : O=C=N-R1, et les régioisomères sont éventuellement séparés par des procédés connus.
